# EUROPEAN PATENT SPECIFICATION

(11) **EP 3 416 659 B1**
(45) Date of publication and mention of the grant of the patent: **05.06.2024**
(21) Application number: 17705400.4
(22) Date of filing: 15.02.2017
(51) Int. Cl.: A61K 35/12, A61K 35/15, A61K 35/17, A61K 35/28, A61K 35/51, A61K 39/00, A61K 39/395, A01N 1/02, C07K 16/28, A61P 7/00, A61P 7/06, A61P 35/02, A61P 37/06

(54) **PREVENTION OF GRAFT REJECTION BY PRIOR USE OF MODIFIED GRAFTS**
PRÄVENTION VON TRANSPLANTATABSTOSSUNG MITTELS VORHERGEHENDER VERWENDUNG VON MODIFIZIERTEN TRANSPLANTATEN
PRÉVENTION DU REJET DE GREFFE PAR UTILISATION PRÉALABLE DES GREFFONS MODIFIÉS

(30) Priority: 15.02.2016 EP 16000373
(43) Date of publication of application: 26.12.2018
(73) Proprietor: Fraunhofer-Gesellschaft zur Förderung der angewandten Forschung e.V., 80686 München (DE)
(72) Inventor: EMMRICH, Frank, 04299 Leipzig (DE); FRICKE, Stephan, 04229 Leipzig (DE); HILGER, Nadja, 04107 Leipzig (DE)
(74) Representative: Simmons & Simmons LLP (Munich)
(86) International application number: PCT/EP2017/053417
(87) International publication number: WO 2017/140735

(56) References cited:
- EP-A1- 2 471 543
- WO-A1-00/38708
- WO-A1-94/26289
- US-A1- 2007 190 052
- BUSHELL A ET AL: "Donor-recipient microchimerism is not required for tolerance induction following recipient pretreatment with donor-specific transfusion and", TRANSPLANTATION, WILLIAMS AND WILKINS, GB, vol. 59, no. 10, 27 May 1995 (1995-05-27), pages 1367-1371, XP009147932, ISSN: 0041-1337, DOI: 10.1097/00007890-199505270-00001
- ZHONGQUAN QI ET AL: "Single dose anti-CD4 monoclonal antibody for induction of tolerance to cardiac allograft in high- and low-responder rat strain combinations", TRANSPLANT IMMUNOLOGY, vol. 5, no. 3, 1 September 1997 (1997-09-01), pages 204-211, XP055137861, ISSN: 0966-3274, DOI: 10.1016/S0966-3274(97)80039-1
- CHRISTIAN BECKER ET AL: "Boosting regulatory T cell function by CD4 stimulation enters the clinic", FRONTIERS IN IMMUNOLOGY, vol. 3, 1 January 2012 (2012-01-01), XP055290387, CH ISSN: 1664-3224, DOI: 10.3389/fimmu.2012.00164
- FRICKE STEPHAN ET AL: "Prevention of graft-versus-host-disease with preserved graft-versus-leukemia-effect by ex vivo and in vivo modulation of CD4+T-cells", CMLS CELLULAR AND MOLECULAR LIFE SCIENCES, BIRKHAUSER VERLAG, HEIDELBERG, DE, vol. 71, no. 11, 26 September 2013 (2013-09-26), pages 2135-2148, XP035317724, ISSN: 1420-682X, DOI: 10.1007/S00018-013-1476-0 [retrieved on 2013-09-26]
- YANG J ET AL: "Amelioration of acute graft-versus-host disease by adoptive transfer of ex vivo expanded human cord blood CD4+CD25+forkhead box protein 3+regulatory T cells is associated with the polarization of Treg/Th17 balance in a mouse model", TRANSFUSION, AMERICAN ASSOCIATION OF BLOOD BANKS, BETHESDA, MD, US, vol. 52, 1 June 2012 (2012-06-01), pages 1333-1347, XP002698696, ISSN: 0041-1132, DOI: 10.1111/J.1537-2995.2011.03448.X

## Description

The invention generally relates to the field of grafts and transplantations thereof and is as specified in the appended claims. In particular, the invention relates to modified grafts, unmodified grafts, or an anti-CD4 antibody as specified in the appended claims for use in methods of treating diseases treatable by transplantation as well as related uses. The said methods generally comprise a step of introducing into a subject a modified graft, and a second step of introducing into said subject an unmodified graft.

The invention relates to the field of solid organ grafts for use in transplantations thereof as specified in the appended claims. The invention relates to modified grafts for use, where introduction thereof is e.g. followed by transplantation of a solid organ, as specified in the appended claims. According to the invention the need for conventionally immunosuppressive drugs may be avoided. Also described are methods of obtaining the modified grafts to be used in the invention. The disclosure relates to modification of allogeneic grafts containing immune competent viable cells by anti human CD4 antibodies before transplantation of solid organs.

### BACKGROUND OF THE INVENTION

In many cases, transplantation of solid organs is still the only curative treatment for many patients with organ dysfunction. Transplantations of solid organs are well established methods in medicine and were performed for circa 50 years [Sayegh, 2004]. The investigation of the blood types A, B, 0 was done by K. Landsteiner in 1901 [Landsteiner, 1901; Durand & Willis, 2010] followed by AB by Alfredo von Castello and Adriano Sturli [de Castello & Sturli, 1902]. The first transplantation of a kidney in animals was performed by Alexis Carrel (1873-1944) in 1906 [Carrel & Guthrie, 1906]. A milestone in the development of solid organ transplantation was the investigation of the tissue compatibility and immune reactions during skin transplantation by Sir Peter Medawar (1915-1987) in 1944 [Medawar, 1944]. The first syngeneic and allogeneic human kidney transplantations were achieved by Joseph E. Murray in 1954 and 1959, respectively [Murray et al., 1976], and the HLA antigens responsible for graft rejection were detected by Jean Dausset in 1958 [Dausset, 1958]. In 1962, the use of the immunosuppressive drug azathioprine permitted the kidney transplantation of a deceased person [Calne et al., 1962]. In Germany, the first (clinical) kidney transplantation between relatives was done by Wilhelm Brosig in 1963 [Eigler, 2002]. First transplantation of pancreas was done by Richard Lillehei in 1966 [Kelly et al., 1967], first successful transplantation of a liver by Tom Starzl in 1967 [Starzl et al., 1968; Starzl et al., 1982]. Also in 1967, the first heart transplantation was performed by Christiaan N. Barnard (1922-2001) in Cape Town, South Africa [Barnard et al., 1967], followed by the first transplantation of heart and lung by Denton A. Cooley in 1969 [Cooley et al., 1969]. For protection of graft rejection, cyclosporin A was first used in 1983 [Kapturczak, 2004]. From the first organ transplantation in 1963 until 2013, 116.650 solid organ transplantations were conducted in Germany [Eurotransplant, 2015a].

In January 2015, 12.500 solid organs were needed, which is more than available [Eurotransplant, 2015b]. Moreover, allogeneic solid organ transplantation can be associated with severe complications. Graft rejection due to genetic disparities, side effects, toxicity, infections and the development of secondary tumors due to life-long treatment with immunosuppressive drugs are serious side effects [Hsu & Katelaris, 2009]. Also the mental impact for the patients due to waiting for a (matching) solid organ graft should be considered [Schulz & Koch, 2005].

Strategies for prophylaxis of graft rejection by conventional immunosuppressive drugs have not been able to distinguish between different T cell clones and therefore not between T cell clones responsible for graft rejection and responsible for maintenance of healthy immunological reactions (e.g., destruction of bacteria, viruses, tumor cells). Generally, the use of conventionally immunosuppressive drugs can lead to suppression of the entire immune system, which enhances the possibility for infections and/or development of malignant tumors [Fricke et al. 2012, 2014].

The investigation of alternative or improved therapeutic approaches or procedures for prevention of graft rejection after solid organ transplantation without conventional immunosuppressive drugs by not ameliorating the desired healthy immunological reactions is still needed.

In a previous work depicted in patent application WO 2012/072268, it was e.g. shown that an *in vitro* method of modifying a graft comprising the steps of a) incubating a graft with an anti-CD4 antibody wherein said incubating is carried out for from 1 min to 7 days and b) removing unbound antibody from said graft, allowed the prevention of Graft-versus-Host-Disease (GvHD) and not decreasing the Graft-versus-Tumor-effect in allogeneic hematopoietic stem cell transplantation at the same time due to induction of immune tolerance. This immune tolerance is considered to be due to modulation of CD4⁺ T cells by MAX.16H5 IgG₁ or CD4.16H5.chimIgG4. CD4 molecules directly bind to constant regions of HLA molecules of antigen presenting cells (APCs) to allow complete T cell activation [In: Murphy KM, Travers P, Walport M., English translation by Seidler L, Haußer-Siller I. (Hrsg): Immunologie, 7. Edition -Heidelberg, Berlin: Spektrum, Akad. Verl. 2008]. To interfere with this binding by non-depleting monoclonal antibodies may inhibit this activation by a total steric blockage, by shortening of cell-cell contact between APC and T cell [cf. Harding, 2002], by induction of negative signals through inhibition of protein tyrosine phosphorylation [June et al., 1990] or induction of T cell anergy [cf. Madrenas, 1996a; Madrenas, 1996b]. EP2471543A1 is a patent family member of WO 2012/072268.

CD4 is a surface glycoprotein primarily expressed on cells of the T lymphocyte lineage including a majority of thymocytes and a subset of peripheral T cells [Harrison, 1993; Luckheeram et al., 2012]. Low levels of CD4 are also expressed by some non-lymphoid cells [Stewart et al., 1986]. On mature T cells, CD4 functions as a co-recognition signal through interaction with MHC Class II molecules expressed on antigen presenting cells [Harrison, 1993]. CD4⁺ T cells primarily constitute the helper subset which regulates T and B cell functions during T-dependent responses to e.g. viral, bacterial, fungal and parasitic infections [Jiang & Dong, 2013]. During the pathogenesis of autoimmune diseases, in particular when tolerance to self-antigens breaks down, CD4⁺ T cells contribute to inflammatory responses which inter alia result in tissue destruction [Bellone, 2005]. These processes are facilitated by the recruitment of inflammatory cells of the hematopoietic lineage, production of antibodies, inflammatory cytokines and mediators, and by the activation of killer cells [Bellone, 2005].

Previously, the murine anti human CD4 monoclonal antibody MAX.16H5 IgG₁ was used in patients with autoimmune diseases or as a protective therapy against transplant rejection [Emmrich, 1991a; Emmrich, 1991b; Reinke, 1991]. In human kidney transplantation, MAX.16H5 IgG₁ had the potential to effectively reduce graft rejection [Reinke, 1991; Reinke, 1994; Reinke, 1995]. The use of MAX. 16H5 IgG₁ not only resulted in suppression of immune activity but also in the induction of tolerance against tetanus toxoid in a triple transgenic mouse model [Laub, 2000; Laub, 2001; Laub, 2002; Strauss, 1994; Fricke, 2014]. The development of these transgenic mice was performed by scientists mentioned in the literature [Laub, 2000; Laub, 2001; Laub, 2002; Strauss, 1994]. CD4/DR3 mice expressing human CD4 and HLA-DR17, a split antigen of HLA-DR3, on a murine CD4-deficient background, were initially bred at the Institute for Laboratory Animal Science, Medical School Hanover (Germany) [Fricke, 2014]. Using these mice, anti human CD4 antibodies can be used directly, and host and donor hematopoiesis could be distinguished by means of human and murine CD4 molecules [Fricke, 2014; Cooke, 1998; O'Connell, 2010; Liu, 2006]. In a T cell activating environment, MAX.16H5 IgG₁ preincubated CD4⁺ T cells were shown to express lower IL-2 mRNA levels and not to activate Lck dependent signal transduction in contrast to controls without prior MAX.16H5 IgG₁ incubation [Fricke, 2014].

Direct application of antibodies to human beings can be associated with an unwanted immune reaction to the therapeutic protein [de Groot & Scott, 2007]. Several mouse monoclonal antibodies have shown promise as therapies in a number of human disease settings but in certain cases have failed due to the induction of significant degrees of a human anti-murine antibody (HAMA) response [Becker, 2012]. The direct application of antibodies to patients may results in severe anaphylactic reactions [de Groot & Scott, 2007].

In previous work, a murine GvHD and tumor transplantation model was shown that allows the direct testing of anti human CD4 antibodies for long-term prevention of GvHD [Fricke, 2014]. However, advantageously, the GvL ("Graft-versus-Leukemia") effect was not decreased [Fricke, 2014]. Without intending to be bound by theory, it may be considered that this effect is CD4 epitope specific [Fricke, 2014]. Advantageously, GvHD was prevented when the graft was short-term pre-incubated (2 hours) with MAX.16H5 IgG₁ and unbound antibodies were removed [Fricke, 2014]. A direct application of the antibodies was not necessary [Fricke, 2014].

However, there still remains a need for methods for introduction of grafts, particularly of non-modified grafts. Also, there still remains a need for advantageous ways of treating diseases treatable by transplantation of grafts. Generally, there still remains a need for promising alternative or improved therapeutic approaches that lack disadvantages of the prior art methodologies.

### SUMMARY OF THE INVENTION

Disclosed is an unmodified graft for use in a method of treating one or more disease(s) treatable by transplantation in a subject, wherein said method comprises a first step of introducing into said subject a modified graft, and a second step of introducing into said subject said unmodified graft, wherein said modified graft is a cell graft containing immune cells, wherein said modification inhibits CD4.

Disclosed is a modified graft for use in a method of treating one or more disease(s) treatable by transplantation, wherein said method comprises a first step of introducing said modified graft into said subject, and a second step of introducing an unmodified graft into said subject, wherein said modified graft is a cell graft containing immune cells, wherein said modification inhibits CD4.

Disclosed is a CD4 antagonist for use in a method of treating one or more disease(s) treatable by transplantation; wherein said method comprises a first step of introducing into said subject a modified graft, and a second step of introducing into said subject an unmodified graft, wherein said modified graft is a cell graft containing immune cells, wherein said modification inhibits CD4.

Disclosed is an unmodified graft, a modified graft and/or a CD4 antagonist for use in i) a surgical transplantation method, ii) a method of inducing transplantation tolerance, wherein a modified graft is used for inducing transplantation tolerance towards an unmodified graft, iii) a method of transferring one or more cell suspension(s), particularly stem cell containing cell suspension(s) from a donor to a subject, iv) a method of transferring one or more tissue(s) from a donor to a subject, v) a method of transferring one or more partial organ(s) from a donor to a subject, vi) a method of transferring one or more organ(s) from a donor to a subject, vii) a method of transferring hematopoietic system reconstituting cells from a donor source into a subject, optionally followed by transferring one or more organ(s) from said donor source into said subject, and/or viii) a method for enhancing immune reconstitution in a subject, optionally followed by transferring one or more organ(s) into said subject; wherein said method comprises a first step of introducing into said subject a modified graft, and a second step of introducing into said subject an unmodified graft, wherein said modified graft is a cell graft containing immune cells, wherein said modification inhibits CD4.

In one aspect, the present invention relates to an unmodified graft for use in a method of treating one or more disease(s) treatable by transplantation in a subject,
wherein said method comprises a first step of introducing into said subject a modified graft, and a second step of introducing into said subject said unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

In a second aspect, the present invention relates to a modified graft for use in a method of treating one or more disease(s) treatable by transplantation,
wherein said method comprises a first step of introducing into said subject said modified graft, and a second step of introducing into said subject an unmodified graft,
wherein said modified graft is a cell suspension graft containing immune cells, that comprises an anti-CD4 antibody, bound to CD4 epitopes of said cell suspension graft, and
wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

In a third aspect, the present invention relates to an anti-CD4 antibody, for use in a method of treating one or more disease(s) treatable by transplantation;
wherein said method comprises a first step of introducing into said subject a modified graft, and a second step of introducing into said subject an unmodified graft,
wherein said modified graft is a cell suspension graft containing immune cells, that comprises an anti-CD4 antibody, bound to CD4 epitopes of said cell suspension graft, and
wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

In a fourth aspect, the present invention relates to an unmodified graft, a modified graft and/or an anti-CD4 antibody, for use in
i) a surgical transplantation method,
ii) a method of inducing transplantation tolerance, wherein a modified graft is used for inducing transplantation tolerance towards an unmodified graft,
iii) a method of transferring one or more cell suspension(s), particularly stem cell containing cell suspension(s) from a donor to a subject,
iv) a method of transferring one or more tissue(s) from a donor to a subject,
v) a method of transferring one or more partial organ(s) from a donor to a subject,
vi) a method of transferring one or more organ(s) from a donor to a subject,
vii) a method of transferring hematopoietic system reconstituting cells from a donor source into a subject, optionally followed by transferring one or more organ(s), from said donor source into said subject,
   or
viii) a method for enhancing immune reconstitution in a subject, optionally followed by transferring one or more organ(s) into said subject;

wherein said method comprises a first step of introducing into said subject a modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft,
and wherein said unmodified graft and said modified graft are allogeneic grafts and comprise immune cells bearing the CD4 antigen.

Generally herein, in preferred embodiments, the modified graft and the unmodified graft are from HLA-related donors.

### SHORT DESCRIPTION OF THE FIGURES

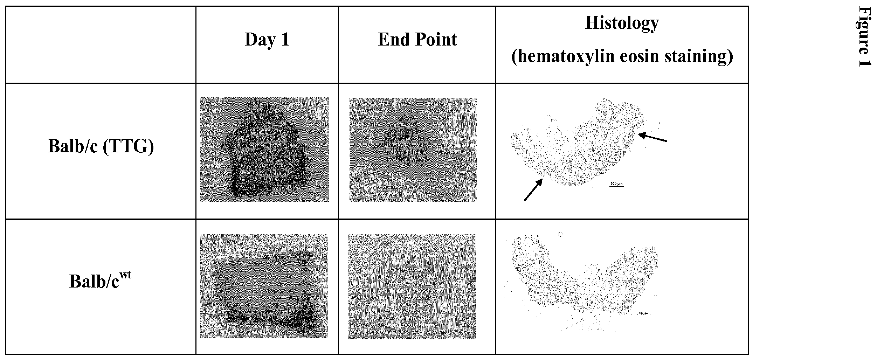

Fig. 1 shows Balb/c (TTG) mice and wildtype Balb/c^{wt} (control) mice 24h and 50 days (d) after transplantation of skin from TTG donor mice. No graft rejection could be observed in Balb/c (TTG) mice.

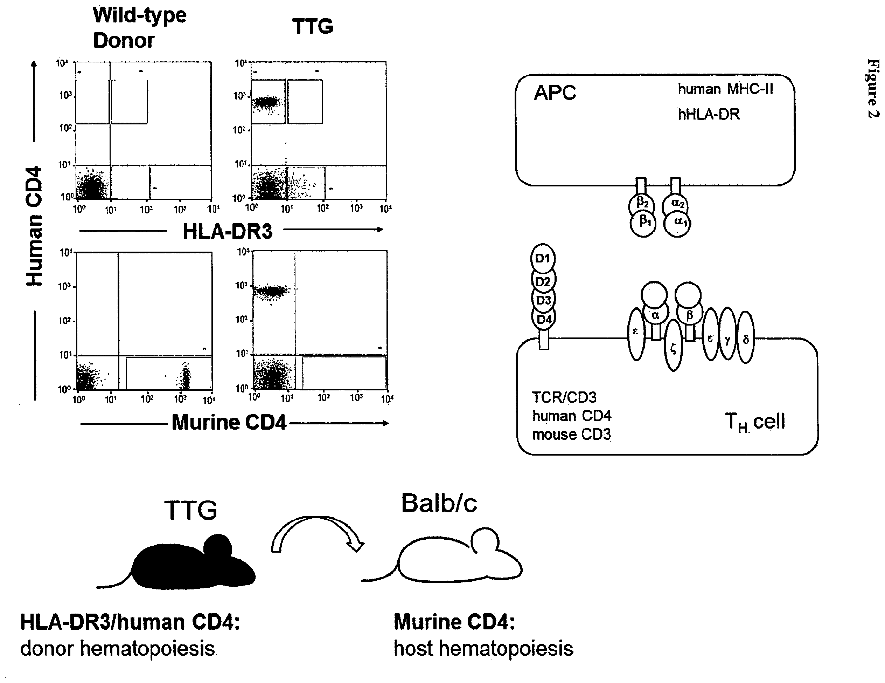

Fig. 2 contains an explanation of C57Bl/6-triple transgenic mice (TTG) as donors. TTG mice express human CD4 and HLA-DR while murine CD4 molecules are knocked out. That allows the determination of specific human surface molecules after transplantation and the direct testing of anti-human CD4 antibodies in mice.

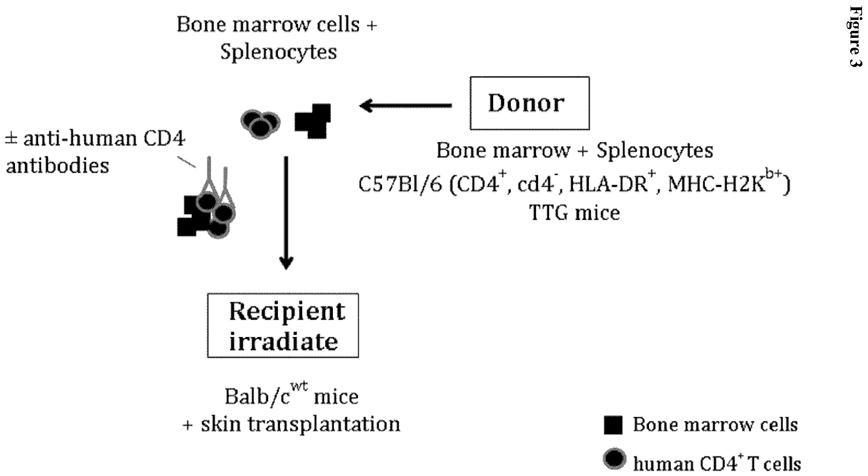

Fig. 3 shows the explanation of an experimental design herein. Bone marrow cells and splenocytes were taken from TTG mice, pooled, incubated in medium containing either anti-human CD4 antibodies or no antibodies for 2 hours, and transplanted in lethally irradiated Balb/c^{wt} mice. The experiment results were compared with results obtained by using donor cells from C57Bl/6 wild-type mice. Therapeutic effects after transplantation were investigated.

Moreover, SEQ ID NOs 1-10 depict the DNA and amino acid sequences of exemplary modified heavy chain variable regions of a particular anti-CD4 antibody for use in the invention.

SEQ ID NOs 11-20 depict the DNA and amino acid sequences of exemplary modified light chain variable regions of a particular anti-CD4 antibody for use in the invention.

### DETAILED DESCRIPTION OF THE PREFERRED EMBODIMENTS

The present invention solves above described objects and overcomes above described deficiencies of prior art methods. It includes the first report showing that *ex vivo* modulation of an allogeneic hematopoietic stem cell graft by anti-human CD4 antibodies MAX.16H5 IgG₁ followed by third party skin transplantation prevents skin graft rejection in a full MHC-mismatch transplantation model.

Without intending to be bound by theory, the present inventors consider that differentiation of or distinction between T cell clones responsible for graft rejection and those responsible for maintenance of healthy immunological reactions could be achieved by modulation of CD4 T-helper cells.

Surprisingly, and further to the previous work described herein above, the present inventors e.g. found that GvHD was not re-induced after single incubation of an allogeneic graft by MAX.16H5 IgG₁ even when cells were transferred in a third party recipient without re-incubation of MAX.16H5 IgG₁. Also, GvL was not prevented. To the inventor's knowledge, such a therapy for induction of tolerance with regard to GvHD by preserving the GvL effect was surprising. Without intending to be bound by theory, the present inventors consider that, advantageously, the maintenance of the immunological effect (e.g. freedom from GvHD) was induced at the day of transplantation and kept up by regulatory T cells.

Among others, the present inventors provide the first report showing that *ex vivo* modulation of an allogeneic hematopoietic stem cell graft by anti human CD4 antibodies MAX.16H5 IgG₁ followed by third party skin transplantation prevents skin graft rejection in a full MHC-mismatch transplantation model.

Currently used drugs for prevention of graft rejection are associated with side effects and do not distinguish between different T cell clones and therefore not between T cell clones responsible for graft rejection and responsible for maintenance of healthy immunological reactions (e.g., destruction of bacteria, viruses, tumor cells) [Fricke, 2014].

Inter alia, anti-human CD4 antibodies MAX.16H5 IgG₁ were tested in murine stem cell transplantation and in a skin transplantation model. *Ex vivo* modified grafts from human CD4⁺ C57Bl/6 mice (cf. Fig. 2) were transplanted into Balb/c^{wt} mice. After 119 days, third party skins from the human CD4⁺ C57Bl/6 mice were transplanted to these recipient mice. The survival rate of these skin grafts was significantly increased in recipients receiving a MAX.16H5 IgG₁ short-term (2 hours) pre-incubated graft (cf. Fig. 1). A direct application of the antibodies to the recipients was not necessary. The anti-human CD4 antibodies MAX.16H5 IgG₁ were long-term effective with regard to survival of solid skin grafts. The nature of the observed induction of immune tolerance by short-term pre-incubation of an antibody with regard to solid organs without the need of conventionally immunosuppressive drugs was advantageous and surprising.

These findings are considered to e.g. make allogeneic organ transplantation safer, and prevent graft rejection due to genetic disparities, reduce side effects, toxicity, and infections as well as the development of secondary tumors due to life-long treatment with immunosuppressive drugs.

Within this patent application, the present inventors could e.g. show that a prior use of a modified graft is suitable to achieve tolerance to an unmodified graft, e.g. in solid organ transplantation (cf. Fig. 3).

Accordingly, the uses of the present invention are e.g. useful i) for reducing the likelihood of donor graft rejection, and organ rejection of a third or higher party organ; ii) for achieving tolerance within the transplanted immunocompetent cells against the third or higher party organ; iii) for achieving tolerance or partial tolerance within the recipient's tissue against the third or higher party organ; and/or iv) for silencing cell activation against a third or higher party organ.

The observed induction of immune tolerance by short-term pre-incubation of an antibody with regard to solid organs without the need for conventional immunosuppressive drugs is advantageous. The present findings are considered to make e.g. allogeneic organ transplantation safer, prevent graft rejection due to genetic disparities, and reduce side effects, toxicity, and infections as well as the development of secondary tumors because life-long treatment with immunosuppressive drugs may be unnecessary.

For obtaining a modified graft, the *in vitro* treatment of cell grafts containing immune cells with antibodies is used, thereby avoiding their direct application *in vivo.* In detail, a short term incubation of a cell suspension (stem cell) graft such as cell suspensions containing T cells, in particular CD4⁺ cells is used, with the aim of tolerance induction or immunosuppression. Without intending to be bound by theory, it is considered that e.g. the anti-CD4 antibody incubation of (stem cell) grafts comprising CD4 positive (immune) cells and subsequent removing of unbound antibodies, preferably removing of only unbound antibodies, results in a modified graft, wherein the antibody labeled cells are not activated, in particular not activated as soon as they encounter specific antigen. Preferably, the antibody labeled cells in the modified graft are anergic cells. Accordingly, e.g. GvHD is not initiated.

Without intending to be bound by theory, it is envisaged that the CD4 antagonist such as anti-CD4 antibody may for example inhibit immune cells (such as lymphocytes) bearing CD4 (e.g. by binding to them) and thereby exerts its beneficial effect. Also, as will be readily apparent to the skilled person, substantially reducing or avoiding the administration of free CD4 antagonists (such as anti-CD4 antibodies, i.e. anti-CD4 antibodies that are not bound to an antigen located on the graft) is advantageous.

In any case, it is considered feasible that CD4 is antagonized by any other way. Such alternative ways of inhibiting CD4 are well-known and easily appliable by the skilled person. CD4 antagonists are well studied and numerous options are known and available to the skilled person - and are e.g. further described herein below.

In general, practicing the present invention is considered to involve one or more of the following advantages: i) no direct application of the CD4 antagonist (such as anti-CD4 antibodies) to the graft recipients is required; ii) suitable use of a short-term incubation of the graft, such as cell suspensions, tissues, and organs containing T cells, in particular CD4⁺ cells; iii) GvHD prevention; iv) prevention of other immunological complications after transplantation of the graft of the invention (e.g. cytokine-release syndrome); v) reduction of costs due to the avoided treatment with conventional immunosuppressive drugs or reduction of their dosage and the significantly reduced amount of antibodies as compared to systemic application; vi) improvement of survival of patients receiving (a) graft(s) in accordance with the invention; vii) facilitation of transplantation of grafts also into patients, into whom regular grafts cannot be transplanted due to expected immunological complications, such as older patients; viii) transplantation of grafts from HLA mismatch donors or grafts from less good HLA matched donors than without the invention; ix) maintenance of the GvL effect; and/or x) facilitation of repeated transplantation of grafts into a subject.

Generally, the present invention relates to subject matter as defined in the appended claims and herein below.

Disclosed is an unmodified graft for use in a method of treating one or more disease(s) treatable by transplantation in a subject, wherein said method comprises a first step of introducing into said subject a modified graft, and a second step of introducing into said subject said unmodified graft, wherein said modified graft is a cell graft containing immune cells, wherein said modification inhibits CD4 (or involves CD4 inhibition, respectively).

Disclosed is a modified graft for use in a method of treating one or more disease(s) treatable by transplantation, wherein said method comprises a first step of introducing into said subject said modified graft, and a second step of introducing into said subject an unmodified graft, wherein said modified graft is a cell graft containing immune cells, wherein said modification inhibits CD4 (or involves CD4 inhibition, respectively).

Disclosed is an CD4 antagonist, preferably an anti-CD4 antibody, for use in a method of treating one or more disease(s) treatable by transplantation; wherein said method comprises a first step of introducing into said subject a modified graft, and a second step of introducing into said subject an unmodified graft, wherein said modified graft is a cell graft containing immune cells, wherein said modification inhibits CD4 (or involves CD4 inhibition, respectively).

That is, in a first aspect, the present invention relates to an unmodified graft for use in a method of treating one or more disease(s) treatable by transplantation in a subject,
wherein said method comprises a first step of introducing into said subject a modified graft, and a second step of introducing into said subject said unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

In a second aspect, the present invention relates to a modified graft for use in a method of treating one or more disease(s) treatable by transplantation,
wherein said method comprises a first step of introducing into said subject said modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

In a third aspect, the present invention relates to an anti-CD4 antibody, for use in a method of treating one or more disease(s) treatable by transplantation;
wherein said method comprises a first step of introducing into said subject a modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

In a fourth aspect, the present invention relates to an unmodified graft, a modified graft and/or an anti-CD4 antibody, for use in
i) a surgical transplantation method,
ii) a method of inducing transplantation tolerance, wherein a modified graft is used for inducing transplantation tolerance towards an unmodified graft,
iii) a method of transferring one or more cell suspension(s), particularly stem cell containing cell suspension(s) from a donor to a subject,
iv) a method of transferring one or more tissue(s) from a donor to a subject,
v) a method of transferring one or more partial organ(s) from a donor to a subject,
vi) a method of transferring one or more organ(s) from a donor to a subject,
vii) a method of transferring hematopoietic system reconstituting cells from a donor source into a subject, optionally followed by transferring one or more organ(s), from said donor source into said subject,
   or
viii) a method for enhancing immune reconstitution in a subject, optionally followed by transferring one or more organ(s) into said subject;

wherein said method comprises a first step of introducing into said subject a modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
   a) incubating a cell suspension graft containing immune cells with the anti-CD4 antibody, wherein said incubating is carried out for from 1 minute to 7 days, and
   b) removing unbound anti-CD4 antibody from said graft,
and wherein said unmodified graft and said modified graft are allogeneic grafts and comprise immune cells bearing the CD4 antigen.

The said modification involves use of the anti-CD4 antibody. The said modification may be effected by the said the anti-CD4 antibody.

Generally herein, the subject may also be referred to as recipient. Preferably herein, the subject is an animal, especially a mammal, particularly a human.

Likewise, preferably herein, the donor(s) (from which the graft(s) is/are isolated and/or derived) is/are an animal, especially a mammal, particularly a human. It is disclosed that the modified and unmodified graft(s) may be from the same donor or from different donors. Preferred embodiments with respect to the donor(s) are defined elsewhere herein.

Preferably, said one or more diseases treatable by transplantation is/are selected from the group consisting of acute myeloid leukemia (AML); acute lymphoid leukemia (ALL); chronic myeloid leukemia (CML); myelodysplastic syndrome (MDS) / myeloproliferative syndrome; malign lymphomas, particularly selected from Morbus Hodgkin, high grade Non-Hodgkin Lymphoma (NHL), mantle cell lymphoma (MCL), low malign NHL, chronic lymphatic leukemia (CLL), multiple myeloma; severe aplastic anemia; thalassemia; sickle cell anemia; immunological defects particularly selected from severe combined immunodeficiency (SCID), Wiskott-Aldrich syndrome (WAS), and hemophagocytic lymphohistiocytosis (HLH); inborn errors of metabolism particularly selected from lysosomal storage disorders and disorders of peroxisomal function; autoimmune diseases; rheumatologic diseases; and recidivisms of any of the above.

Even more preferably, said one or more diseases are one or more hematological malignancies especially selected from acute myeloid leukemia (AML); acute lymphoid leukemia (ALL); chronic myeloid leukemia (CML); myelodysplastic syndrome (MDS) / myeloproliferative syndrome; malign lymphomas, particularly selected from Morbus Hodgkin, high grade Non-Hodgkin lymphoma (NHL), mantle cell lymphoma (MCL), low malign Non-Hodgkin lymphoma (NHL), chronic lymphatic leukemia (CLL), multiple myeloma; severe aplastic anemia; thalassemia; and sickle cell anemia.

In certain embodiments herein, the disease treatable by transplantation is any condition requiring transplantation.

In certain preferred embodiments herein, the disease treatable by transplantation is a disease involving organ malfunction. Accordingly, said disease may e.g. require transplantation to replace an organ, which does not function as desired.

In certain embodiments herein, the disease treatable by transplantation is transplant rejection, such as organ rejection. Further examples are GvHD and donor graft rejection. That is to say, in the latter cases, a previously transplanted transplant, such as an organ, may be replaced by another transplant, such as an organ.

Generally herein, said one or more diseases treatable by transplantation also include recidivisms of any of the above as well as any combination of diseases mentioned herein.

As used herein, a "cell graft containing immune cells" essentially refers to a graft comprising immune cells. Generally, the cell graft containing immune cells is not particularly limited, with particular embodiments corresponding to those described herein below, e.g. in context with the modified graft.

Generally, grafts, and also cell grafts containing immune cells, are very well known to the person skilled in the art. Also the use of grafts including cell grafts containing immune cells in transplantation is well known in the art. The graft may comprise a cell suspension, a tissue and/or an organ. The graft may be a cell suspension, a tissue and/or an organ. The grafts may be selected from the group consisting of a cell suspension, a tissue and an organ. Generally, herein, a graft may also be a combination of grafts, such as a combination of one or more of the grafts referred to above, e.g. a combination of an organ and a cell suspension. The graft is not an artificial graft. It is understood that grafts, and also cell grafts containing immune cells, may be intended for and useful for therapeutic purposes *in vivo,* in particular intended for and useful for treating a disease involving organ malfunction. Said disease may e.g. require transplantation to replace an organ, which does not function as desired. Accordingly, grafts, and also cell grafts containing immune cells, may exclude cells and compositions comprising cells, which are intended for and/or useful only for mechanistic studies in animal models, which do not aim at a therapy of a disease.

It is disclosed that each of the said grafts is selected from the group consisting of a cell suspension, a tissue and an organ. In the present invention, the modified graft may be selected from the group consisting of a cell suspension, a tissue and an organ. In the present invention, the unmodified graft is selected from the group consisting of a tissue and an organ. In particular embodiments, said modified graft is a cell suspension and said graft is a cell suspension. In particular embodiments, said modified graft is a cell suspension and said graft is a tissue. In particular embodiments, said modified graft is a cell suspension and said graft is an organ.

In some particular embodiments the graft does not include embryonic stem cells that are not derived and/or reprogrammed from adult stem cells. Hence, in some particular embodiments any embryonic stem cells included in the graft are embryonic stem cells that are derived and/or reprogrammed from adult stem cells. The grafts do not consist of or do not comprise totipotent human embryonic stem cells. The grafts do not consist of or do not comprise human embryonic stem cells. The grafts do not comprise embryonic stem cells derived from a human embryo.

As used herein, an unmodified graft is a regular graft as it is well-known to a skilled person. In particular, it will be readily understood by the skilled person that such unmodified graft is not modified as the modified graft described herein, i.e. does not comprise the modifications of the modified graft herein. Accordingly, the unmodified graft may be described as not having been modified by use of a CD4 antagonist. Accordingly, the unmodified graft may be described as not comprising CD4 antagonists (such as anti-CD4 antibodies). Accordingly, the unmodified graft may be described as not comprising a modification that inhibits CD4. Preferred particular embodiments of an unmodified graft are described herein below.

The present invention advantageously uses a modified graft. Generally, modified grafts used in the invention may be used as it is known in the art for unmodified grafts, e.g. as regards their introduction (e.g. transplantation) into a subject.

As used herein, a modified graft is a graft that has been modified (preferably that has artificially been modified), by an *in vitro* method described herein, by use of an anti-CD4 antibody. Hence, said modification may be said to involve use of an anti-CD4 antibody. Likewise, said modification may be said to be effected by an anti-CD4 antibody. The modified graft herein may have been modified prior to the uses of the invention or during the uses of the invention.

Generally herein, the modification of the modified graft is said to inhibit CD4. Hence, the modified graft may be described as comprising a modification that inhibits CD4. Accordingly, it may be said that the modified graft herein has been / is modified to inhibit CD4.

Generally, inhibition as referred to herein may not mean absolute inhibition but also includes partial inhibition. Sufficient degrees of inhibition may readily be determined by the skilled person. Particular degrees of inhibition e.g. correspond to e.g. the percentage values recited in context with antibody binding to a graft of the modified graft described elsewhere herein. A particular degree of inhibition may achieve at least one of the advantageous effects disclosed herein.

As used herein, a "modified graft wherein the modification inhibits CD4" ("... involves CD4 inhibition", or other suchlike and/or equivalent terms) may refer to a modified graft, where the expression and/or function, such as the function, of CD4 is inhibited (or impaired). Ways to inhibit CD4 are known to the skilled person and are not particularly limited. They may involve use of (or be effected by, respectively) any CD4 antagonist(s), and particularly of any antagonist(s) of CD4 described herein. In a disclosure, a CD4 antagonist (or e.g. also a mixture of CD4 antagonists) may be used.

As the skilled reader will readily appreciate, the term "wherein the modification inhibits CD4" and suchlike terms is/are replaced by the term "wherein the modification inhibits cellular functions via CD4". The term "wherein the modification inhibits CD4" and suchlike terms may be replaced by the term "wherein the modification inhibits or impairs cellular functions downstream of CD4, in particular binding and/or recruitment". Cellular functions that may be inhibited via CD4 are readily known to the skilled person, particularly in view of his knowledge on CD4 and its mode of action in the cell. At least one such cellular function may be inhibited.

Likewise, the term "wherein the modification involves CD4 inhibition" and suchlike terms may be replaced by the term "wherein the modification involves CD4-mediated inhibition of function". The term "wherein the modification involves CD4 inhibition" and suchlike terms may be replaced by the term "wherein the modification involves inhibition or impairment of function". The term "wherein the modification involves CD4 inhibition" and suchlike terms may be replaced by the term "wherein the modification involves inhibition or impairment of CD4-mediated function"
Generally, it is disclosed that CD4 antagonists to be used in context with the present disclosure include CD4 antagonists, which modulate CD4 expression and/or function. Accordingly, in a disclosure, a CD4 antagonist herein may modulate, particularly inhibit, expression of CD4. Accordingly, in a disclosure, a CD4 antagonist herein may modulate, particularly inhibit, the function of CD4 - especially by binding to it. Accordingly, the CD4 antagonists herein may be CD4 ligands. Generally, CD4 antagonists such as CD4 inhibitors or CD4 ligands, respectively, are known in the art. Numerous CD4 antagonists, and particularly CD4 ligands that bind to CD4, are commercially available.

CD4 antagonists are generally well known in the art. Non-limiting examples include the ones described herein. Generally herein, said CD4 antagonists, e.g. said CD4 inhibitors or CD4 ligands, respectively, may be extra- or intracellular agents (such as ligands). Generally herein, said CD4 inhibition may directly or indirectly be caused by said CD4 antagonist.

In a disclosure herein, the CD4 antagonist binds to CD4.

In the invention, the CD4 antagonist is an anti-CD4 antibody. Anti-CD4 antibodies are generally well-known in the art. Numerous anti-CD4 antibodies are commercially available.

Antibodies and also anti-CD4 antibodies are well known in the art. As used herein, by "antibody" is meant inter alia a protein of the immunoglobulin family that is capable of specifically combining, interacting or otherwise associating with an antigen, wherein said combining, interacting or otherwise associating (such as binding) of the antibody to the antigen is mediated by complementarity-determining regions (CDRs). Similarly, term "antigen" is used herein to refer to a substance that is capable of specifically combining, interacting or otherwise associating with said antibody. In the context of the anti-CD4 antibody of the present invention the antigen is meant to be CD4, particularly human CD4. As used herein, the term "CDR" refers to the "complementarity-determining region" of an antibody, i.e. to one of the hypervariable regions within an immunoglobulin variable domain contributing to the determination of antibody specificity. CDRs are well known to a person skilled in the art. Typically, both the heavy chain immunoglobulin variable domain and the light chain immunoglobulin variable domain contain three CDRs. In the context of the present invention, the term "antibody" is considered to also relate to antibody fragments including for example Fv, Fab, Fab' and F(ab')₂ fragments. Such fragments may be prepared by standard methods [for example; Coligan et al., 1991-1997]. The present invention also contemplates the various recombinant forms of antibody derived molecular species well known in the art. Such species include stabilized Fv fragments including single chain Fv forms (e.g., scFv) comprising a peptide linker joining the VH and VL domains, or an Fv stabilized by interchain disulphide linkage (dsFv) and which contain additional cysteine residues engineered to facilitate the conjoining of the VH and VL domains. Equally, other compositions are familiar in the art and could include species referred to as "minibodies"; and single variable domain "dAbs". Other species still may incorporate means for increasing the valency of the modified antibody V-region domain, i.e. species having multiple antigen binding sites for example by engineering dimerization domains (e.g., "leucine zippers") or also chemical modification strategies. Moreover, the term "antibody" also relates to multimers of scFv such as diabodies, triabodies or tetrabodies, tandabs, flexibodies, bispecific antibodies, and chimeric antibodies, all known in the art. As used herein, antibodies are considered to also include any bivalent or multivalent antibodies. They also include any antibody derivatives and any other derivatives known to the skilled person. In some embodiments, the antibody is a polyclonal antibody. In preferred embodiments, the antibody is a monoclonal antibody. Further embodiments of "antibody" may be taken from WO 2012/072268.

According to the invention, the term "anti-CD4 antibody" refers to an antibody, which has the ability to bind to CD4. Preferably herein, the anti-CD4 antibody is an anti human CD4 antibody. "CD4" or "cluster of differentiation 4" refers to a protein, more precisely a surface glycoprotein, well known to the person skilled in the art [cf. above, cf. also Bowers et al., 1997]. In the present context CD4 may also refer to a fragment of full-length CD4, or an otherwise modified form of CD4, provided that the fragment or otherwise modified form still functions as an antigen in the context of the antibody of the present invention.

Particularly preferred examples of anti-CD4 antibodies that may be used in accordance with the present invention are described elsewhere herein.

Non-limiting (further) examples of CD4 antagonists are peptide ligands (including naturally occurring peptide ligands and peptide constructs).

Certain further disclosure includes aptamers. In a further disclosure, the CD4 antagonist is cyclo(CNSNQIC). In a further disclosure, the CD4 antagonist is 4,4'-diisothiocyano-2,2'-dihydrostilbenedisulfonic acid.

Generally, the modified graft comprises an anti-CD4 antibody.

In further aspects herein, which are related to the first, second and third aspects, said use(s) in a method comprise(s) a step of introducing into said subject an unmodified graft, wherein said subject has previously been introduced with a modified graft, wherein the modified graft is a cell suspension graft containing immune cells, that comprises an anti-CD4 antibody bound to CD4 epitopes of said cell suspension graft. Said method(s) comprise(s) a step of introducing into said subject an unmodified graft, wherein a modified graft has previously been introduced into said subject, wherein the modified graft is a cell suspension graft containing immune cells, that comprises an anti-CD4 antibody bound to CD4 epitopes of said cell suspension graft. Generally, preferred embodiments of those related aspect correspond to those of the respective aspects as described herein.

In a fourth aspect herein, the present invention relates to an unmodified graft, a modified graft and/or an anti-CD4 antibody for use in any one or more method(s) of the group consisting of i) a surgical transplantation method, ii) a method of inducing transplantation tolerance, wherein a modified graft is used for inducing transplantation tolerance to an unmodified graft, iii) a method of transferring one or more cell suspension(s), particularly stem cell containing cell suspension(s) from a donor to a subject, iv) a method of transferring one or more tissue(s) from a donor to a subject, v) a method of transferring one or more partial organ(s) from a donor to a subject, vi) a method of transferring one or more organ(s) from a donor to a subject, vii) a method of transferring hematopoietic system reconstituting cells from a donor source into a subject, optionally followed by transferring one or more organ(s), from said donor source into said subject, and viii) a method for enhancing immune reconstitution in a subject, optionally followed by transferring one or more organ(s) into said subject.

Generally, the above method(s) preferably comprise(s) a first step of introducing into said subject a modified graft, and a second step of introducing into said subject an unmodified graft, wherein said modified graft is a cell suspension graft containing immune cells, that comprises an anti-CD4 antibody bound to CD4 epitopes of said cell suspension graft.

Alternatively, in a further disclosed variant, said method(s) comprise(s) a step of introducing into said subject an unmodified graft, wherein said subject has previously been introduced with a modified graft, wherein the modified graft is a cell graft containing immune cells, wherein said modification inhibits CD4 (or involves CD4 inhibition, respectively).

In detail, in one particular embodiment the fourth aspect relates to an unmodified graft, a modified graft and/or an anti-CD4 antibody for use in a surgical transplantation method.

In one particular embodiment the fourth aspect relates to an unmodified graft, a modified graft and/or an anti-CD4 antibody for use in a method of inducing transplantation tolerance, wherein a modified graft is used for inducing transplantation tolerance to an unmodified graft.

In one particular embodiment the fourth aspect relates to an unmodified graft, a modified graft and/or an anti-CD4 antibody for use in a method of transferring one or more cell suspension(s), particularly stem cell containing cell suspension(s) from a donor to a subj ect.

In one particular embodiment the fourth aspect relates to an unmodified graft, a modified graft and/or an anti-CD4 antibody for use in a method of transferring one or more tissue(s) from a donor to a subject.

In one particular embodiment the fourth aspect relates to an unmodified graft, a modified graft and/or an anti-CD4 antibody for use in a method of transferring one or more partial organ(s) from a donor to a subject.

In one particular embodiment the fourth aspect relates to an unmodified graft, a modified graft and/or an anti-CD4 antibody for use in a method of transferring one or more organ(s) from a donor to a subject.

In one particular embodiment the fourth aspect relates to an unmodified graft, a modified graft and/or an anti-CD4 antibody for use in a method of transferring hematopoietic system reconstituting cells from a donor source into a subject, optionally followed by transferring one or more organ(s), from said donor source into said subject.

In one particular embodiment the fourth aspect relates to an unmodified graft, a modified graft and/or an anti-CD4 antibody for use in a method for enhancing immune reconstitution in a subject, optionally followed by transferring one or more organ(s) into said subject.

In certain embodiments, the fourth aspect relates to an unmodified graft for use in any of said method(s). In certain embodiments, the fourth aspect relates to a modified graft for use in any of said method(s). In certain embodiments, the fourth aspect relates to an anti-CD4 antibody for use in any of said method(s).

Generally herein, embodiments of the fourth aspect (and the said variant related thereto) correspond to embodiments of the other aspects described herein.

Generally herein, the first, second, third and fourth aspects herein, the said first step recited in context with the respective method(s) is carried out before said second step recited in context with the said method(s).

In particular embodiments of the first, second, third and fourth aspects herein, said first step is carried out from 30 min to 50 years, particularly from one hour to 10 years, such as to 9, 8, 7, 6, 5, 4, 3, or two years, such as from one day to 1 year, such as to 11, 10, 9, 8, 7, 6, 5, 4, 3, or two months before said second step. In certain embodiments, said first step is carried out from one hour to two months before said second step. In certain embodiments, said first step is carried out at least one hour, particularly at least three weeks, before said second step.

In particular embodiments, said first step is carried out at least one hour, particularly at least one day, particularly at least one month, particularly at least one year before said second step. In particular embodiments herein, said first step is carried out at least 12 hours, particularly at least one day, particularly at least one week, particularly at least three weeks, particularly at least six months before said second step.

In particular embodiments, said first step is carried out up to 30 years, particularly up to 10 years, particularly up to 1 year, particularly up to 1 month before said second step. In particular embodiments, said first step is carried out up to 20 years, particularly up to 5 years, particularly up to 2 years, particularly up to 6 months, particularly up to 12 weeks before said second step.

Similarly, where the said uses in method(s) herein are defined as comprising a step of introducing into said subject an unmodified graft, wherein said subject has previously been introduced with a modified graft, the timing of the previous introduction is preferably further defined as detailed above. Preferably, said method(s) herein are defined as comprising a step of introducing into said subject an unmodified graft, wherein a modified graft has previously been introduced into said subject, the timing of the previous introduction is preferably further defined as detailed above.

Generally, in preferred embodiments of the first, second, third and fourth aspects herein, said modified graft comprises stem cells.

It is disclosed that the modified graft is a cell graft containing immune cells that has been modified by a CD4 antagonist, particularly wherein said modification inhibits CD4.

Generally, in a disclosure, the modified graft, particularly a cell graft containing immune cells, comprises a CD4 antagonist.

In the invention, the modified graft is a cell suspension graft containing immune cells that comprises an anti-CD4 antibody bound to CD4 epitopes thereof.

In certain preferred embodiments of the invention the modified graft, the modified graft is a cell suspension graft containing immune cells that comprises an anti-CD4 antibody bound to from 40% to 100% of the CD4 epitopes thereof. Such graft is obtainable by the *in vitro* method disclosed herein.

Preferably, the modified cell graft containing immune cells, comprises anti-CD4 antibodies bound to from 50% to 100%, particularly 60% to 100%, particularly 70% to 100%, more particularly 80% to 100%, more particularly 90% to 100%, more particularly 95% to 100%, more particularly 99% to 100%, of the accessible CD4 epitopes of said graft. Most preferably, essentially all of the accessible CD4 epitopes of the cell graft containing immune cells are bound to anti-CD4 antibodies.

Said modified graft is obtained by or obtainable by an *in vitro* method comprising the step a) incubating a cell suspension graft containing immune cells with an anti-CD4 antibody, wherein said incubating is carried out for from 1 minute to 7 days, and step b) removing unbound antibody from said graft, thereby reducing the amount of unbound antibody in said graft.

In a more preferred embodiment, the amount of unbound antibody in said graft is reduced by at least 30%, 40%, 50%, 60%, 70%, 80%, 90%, 95% or 99%, or is reduced by 100%.

Said modified graft may be obtained by or obtainable by an *in vitro* method comprising the step a) incubating a cell suspension graft containing immune cells with an anti-CD4 antibody, wherein said incubating is carried out for from 1 minute to 7 days, and optionally comprising the step b) removing unbound antibody from said graft by washing the modified graft at least one time or at least 2 times, such as 2, 3, 4, 5 or 6 times. By washing the modified graft, the amount of unbound antibody in said graft is reduced. In a more preferred embodiment, the amount of unbound antibody in said graft is reduced by at least 30%, 40%, 50%, 60%, 70%, 80%, 90%, 95% or 99%, or is reduced by 100%. Suitable washing liquids are known to a skilled person and include buffered saline solutions.

It is understood that a certain amount of CD4-positive immune cells may be lost or removed unspecifically during the washing step(s). The modified graft contains immune cells, in particular CD4-positive immune cells, such as CD4⁺-T cells, to which anti-CD4 antibody is bound. In a more preferred embodiment, the modified graft contains at least 10%, 20%, 30%, 40%, 50%, 60%, 70%, 80%, 90% or 95% of the CD4-positive immune cells of the cell graft containing immune cells prior to incubation.

Said modified graft is obtained by or obtainable by, respectively, an *in vitro* method comprising the step a) incubating a cell suspension graft containing immune cells with an anti-CD4 antibody, wherein said incubating is carried out for from 1 minute to 7 days, and the step b) removing only unbound antibody from said graft, thereby reducing the amount of unbound antibody in said graft. Accordingly, the modified cell graft containing immune cells is obtainable in accordance with an *in vitro* method described herein.

As used herein, an *"in vitro* method" refers to a method that is performed outside a living subject. It particularly also includes an *"ex vivo* method", such as in case of the graft comprising or being a tissue or an organ, but particularly excludes an *"in vivo* method" performed inside a living subject.

As used herein, "unbound antibody" refers to an antibody which, following the step of incubating, is not bound to the graft. In other words, it refers to an antibody which is not essentially associated with its ligands on the graft.

Said *in vitro* method to be used in the invention may be a method of modifying a cell graft containing immune cells that comprises the steps of a) incubating a cell graft containing immune cells with an anti-CD4 antibody, wherein said incubating is carried out for from 1 min to 7 days, b) removing unbound antibody from said graft. Preferably, said incubating in step a) of said *in vitro* method is carried out for from 1 min to 1 day.

Said *in vitro* method to be used in the invention may is method of modifying a cell suspension graft containing immune cells that comprises the steps of a) incubating a cell suspension graft containing immune cells with an anti-CD4 antibody, especially wherein said incubating is carried out for from 1 min to 7 days, b) removing unbound antibody from said graft, thereby reducing the amount of unbound antibody in said graft. In a more preferred embodiment, the amount of unbound antibody in said graft is reduced by at least 30%, 40%, 50%, 60%, 70%, 80%, 90%, 95% or 99%, or is reduced by 100%. Preferably, said incubating in step a) of said *in vitro* method is carried out for from 1 min to 1 day.

In another preferred embodiment, said *in vitro* method to be used may be a method of modifying a cell suspension graft containing immune cells that comprises the steps of a) incubating a cell suspension graft containing immune cells with an anti-CD4 antibody, especially wherein said incubating is carried out for from 1 min to 7 days, b) removing unbound antibody from said graft, by washing the modified graft at least one time or at least 2 times, such as 2, 3, 4, 5 or 6 times. By washing the modified graft, the amount of unbound antibody in said graft is reduced. In a more preferred embodiment, the amount of unbound antibody in said graft is reduced by at least 30%, 40%, 50%, 60%, 70%, 80%, 90%, 95% or 99%, or is reduced by 100%. Preferably, said incubating in step a) of said *in vitro* method is carried out for from 1 min to 1 day.

In a preferred embodiment, it is understood that a certain amount of CD4-positive immune cells may be lost or removed unspecifically during the washing step(s). In a yet further preferred embodiment, the modified graft obtained by the method contains immune cells, in particular CD4-positive immune cells, such as CD4⁺-T cells, to which anti-CD4 antibody is bound. In a more preferred embodiment, the modified graft contains at least 10%, 20%, 30%, 40%, 50%, 60%, 70%, 80%, 90% or 95% of the CD4-positive immune cells of the cell graft containing immune cells prior to incubation.

In another preferred embodiment, said *in vitro* method to be used in the invention may be a method of modifying a cell suspension graft containing immune cells that comprises the steps of a) incubating a cell suspension graft containing immune cells with an anti-CD4 antibody, wherein said incubating is carried out for from 1 min to 7 days, b) removing only unbound antibody from said graft. Preferably, said incubating in step a) of said *in vitro* method is carried out for from 1 min to 1 day.

Generally, preferred embodiments for said *in vitro* methods may be taken from WO 2012/072268.

In step a) of the *in vitro* method used herein, the (step of) incubating may be carried out for a time sufficient to allow binding of said antibody to said graft. Preferably, said incubating is carried out for a time sufficient to allow the binding of anti-CD4 antibodies to from 40% to 100%, particularly 50% to 100%, particularly 60% to 100%, particularly 70% to 100%, more particularly 80% to 100%, more particularly 90% to 100%, more particularly 95% to 100%, more particularly 99% to 100%, of the accessible CD4 epitopes of said graft. Most preferably, following said incubating, anti-CD4 antibodies bind to essentially all of the accessible CD4 epitopes of said graft. In a preferred embodiment, it is understood that the accessible CD4 epitopes or CD4-positive immune cells (or immune cells bearing the CD4 antigen) of said graft are not removed or substantially not removed. Accordingly, in a further preferred embodiment, immune cells bearing the CD4 antigen are not depleted, or not substantially depleted from the unmodified graft. The modified graft obtained by or obtainable by the *in vitro* method comprises immune cells bearing the CD4 antigen of the unmodified graft.

An appropriate incubation period will easily be determined by the person skilled in the art. Usually, an appropriate incubation period will depend on the type of graft used. A preferred incubation period may also dependent on the amount of antibody used. Generally, as the graft is a cell suspension, shorter incubation periods will be required than where the graft is e.g. an organ. Generally, where the graft comprises or is a tissue or an organ, longer incubation periods may be used to allow the antibody to be transported - e.g. via diffusion - into the respective compartments.

Moreover, in any case, the skilled person may easily test the (status of the) binding of the anti-CD4 antibodies according to methods well known within the art that may, for example, involve flow cytometry.

Said incubating in step a) of said *in vitro* method is carried out for from 1 min to 7 days. Preferably, said incubating in step a) of said *in vitro* method is carried out for from 1 min to 1 day.

Generally, short incubation periods are preferred herein over long incubation periods in order to minimize any possible damage to the graft due to *in vitro* processing.

Particularly, said incubating may be carried out for from 1 to 150 min, particularly for from 5 min to 150 min, more particularly for from 10 min to 150 min, more particularly for from 30 min to 150 min, more particularly for from 40 minutes to 120 min, more particularly for from 45 min to 90 min, especially for from 50 min to 70 min.

In alternative embodiments, said incubating may be carried out for from 150 min to 7 days, particularly for from 150 min to 5 days, more particularly from 150 min to 3 days, more particularly from 150 min to 1 day, especially for from 150 min to 8 hours. In a further preferred embodiment, incubating may be carried out for from 1 min to 1 day.

As to the "removing" of unbound (anti-CD4) antibody in accordance with e.g. step b) of the *in vitro* method used herein, various ways of performing said step are known to the skilled person. One exemplary way of removing unbound antibody from the graft is by washing the graft. Washing may e.g. occur by employing centrifugation where the graft comprises or is a cell suspension. In one preferred embodiment, the amount of unbound antibody in said graft is reduced by washing the graft. In a more preferred embodiment, the amount of unbound antibody in said graft is reduced by at least 30%, 40%, 50%, 60%, 70%, 80%, 90%, 95% or 99%, or is reduced by 100%. Preferably, only unbound (anti-CD4) antibody is removed in accordance with e.g. step b) of the *in vitro* method used herein. A few CD4-positive immune cells may be lost unspecifically during removal of unbound antibodies, in particular be washing. In a preferred embodiment, the modified graft contains at least 10%, 20%, 30%, 40%, 50%, 60%, 70%, 80%, 90% or 95% of the CD4-positive immune cells of the cell graft containing immune cells prior to incubation.

In said step, preferably at least 40%, more particularly at least 50%, more particularly at least 60%, more particularly at least 70%, more particularly at least 80%, more particularly at least 90%, of unbound (anti-CD4) antibody are removed from the graft. Preferably, up to 100% of unbound (anti-CD4) antibody are removed from the graft.

The amount of antibody employed in the above step of incubating is not particularly limited. Appropriate amounts may easily be determined by the person skilled in the art and may depend on e.g. the type of graft used. Preferably according to the invention, said incubating is carried out with an antibody amount of from 0.1µg to 100mg. In a preferred amount, Preferably, in accordance with the invention, particularly in case of the graft being a cell suspension, an amount of from 2 × 10⁶ cells to 2 × 10¹⁰ nucleated cells, particularly of from 4 × 10⁶ to 1 × 10⁹ nucleated cells, more particularly of from 1 × 10⁷ to 1 × 10⁸ nucleated cells are administered to said subject, preferably to the human subject. accordingly, in a yet further embodiment, said incubating is carried out with an antibody amount of from 0.1µg/1 × 10⁹ nucleated cells to 100mg/2 × 10⁶ nucleated cells, more preferably 0.1µg/1 × 10⁹ nucleated cells to 100mg/2 × 10⁷ nucleated cells.

In particular embodiments said incubating in step a) of said *in vitro* method is carried out with an antibody amount of from 0.1µg/ml to 10mg/ml.

In certain embodiments, where the graft is a cell suspension, said incubating is carried out with an antibody concentration of from 0.1µg/ml cell suspension to 150µg/ml cell suspension, particularly from 7µg/ml cell suspension to 100µg/ml cell suspension, more particularly from 30µg/ml cell suspension to 100µg/ml cell suspension, especially from 40µg to 60µg/ml cell suspension.

In some disclosure, where the graft is a tissue or where the graft is an organ, said incubating is carried out with an antibody amount of from 0.1mg to 10mg, particularly from 1mg to 10mg, more particularly from 2mg to 9mg, more particularly from 3mg to 8mg, especially from 4mg to 6mg.

In some disclosure, where the graft is a tissue or where the graft is an organ, said incubating is carried out with an antibody concentration in the incubation solution of from 0.1mg/ml to 10mg/ml, particularly from 1mg/ml to 10mg/ml, more particularly from 2mg/ml to 9mg/ml, more particularly from 3mg/ml to 8mg/ml, especially from 4mg/ml to 6mg/ml. The specified volume may include the volume of said tissue or organ as well as the volume of the (antibody-containing) solution, in which said tissue or organ is incubated.

In some disclosure, where the graft is a tissue or where the graft is an organ, said incubating is carried out by incubating said tissue or organ in a solution having an antibody concentration of from 10µg/ml to 150µg/ml, particularly from 20µg/ml to 100µg/ml, more particularly from 30µg/ml to 100µg/ml, especially from 40µg/ml to 60µg/ml. The specified volume may include the volume of said tissue or organ as well as the volume of the (antibody-containing) solution, in which said tissue or organ is incubated.

When incubating tissues and/or organs with a CD4 antagonist (e.g. an anti-CD4 antibody-containing solution), the skilled person may readily perform such incubation e.g. by means of a suitable container.

In any case, the selection of suitable amounts of CD4 antagonist, such as of anti-CD4 antibody, is well within the expertise of the skilled person. Generally, higher amounts or concentrations, respectively, of antagonist (e.g. antibody) may be used where the graft comprises or is a tissue or an organ. Moreover, the selection of an exact amount or a concentration, respectively, of antagonist (e.g. antibody) used will also depend on the size of such tissue or organ.

In the following, further particular embodiments of the modified graft to be used in the invention are described: In some preferred embodiments of the invention, the modified graft comprises stem cells. A graft comprising stem cells may also be referred to herein as a stem cell graft.

According to the present invention, the modified graft comprises immune cells bearing the CD4 antigen. Such cells are well known to the person skilled in the art. In certain preferred embodiments, these immune cells are CD4 positive T lymphocytes or precursor cells thereof. In certain preferred embodiments, these immune cells include, but are not limited to T helper cells and cells belonging to the monocyte and macrophage lineage, such as monocytes and macrophages. Another example for such cells are microglia.

In some disclosure, the modified graft comprises, or is, a tissue, such as a stem-cell-containing tissue. Suitable tissues may include blood, muscle, adipose tissue, connective tissue, epithelium, embryonic, and cellular tissue.

In some disclosure, the modified graft comprises, or is, an organ, such as a stem-cell-containing organ. Suitable organs include skin, intestine, kidney, and liver. Preferably, said organ is an intestine.

The modified graft is a cell suspension, preferably a stem-cell-containing cell suspension. Suitable cell suspensions and methods for obtaining them are well known to the skilled person. For example, a cell suspension graft may be obtained by puncture of bones comprising bone marrow, e.g. puncture of the iliac crests or sterna or taken from stem cell niches throughout the whole body, e.g. fat tissue, tooth root, root of a hair and any other source mentioned above.

In preferred embodiments, the modified graft, which is cell suspension, comprises T cells, monocytes and macrophages. In another preferred embodiment, the modified graft is a cell suspension that comprises T cells and non-immune cells, such as stem cells. In a disclosure, the stem-cell-containing tissue, the stem-cell-containing organ, or the stem-cell-containing cell suspension, comprises immune cells, more preferably immune cells bearing the CD4 antigen, in particular CD4 positive T lymphocytes or precursor cells thereof.

The modified graft, comprises immune cells bearing the CD4 antigen, in particular CD4 positive T lymphocytes or precursor cells thereof.

In certain preferred embodiments the modified graft, which is a cell suspension, comprises any of bone marrow stem cells, peripheral blood stem cells, umbilical cord blood stem cells, adult stem cells of the bone marrow such as NA-BMCs, embryonic stem cells (particularly derived and/or reprogrammed from adult stem cells), and any pluripotent stem cells, wherein the latter includes induced pluripotent cells, such as cells derived and/or reprogrammed from adult stem cells (i.e. including pluripotent embryonic stem cells).

In preferred embodiments, the modified graft is a bone marrow suspension, particularly comprising bone marrow stem cells. Generally, the modified graft, particularly the bone marrow suspension, may additionally comprise any of stem cells comprised in blood cells, cord blood cells, donor lymphocytes, peripheral blood stem cells, adult stem cells of the bone marrow, embryonic stem cells (particularly derived and/or reprogrammed from adult stem cells), and any pluripotent stem cells, wherein the latter includes induced pluripotent cells, such as cells derived and/or reprogrammed from adult stem cells (i.e. including pluripotent embryonic stem cells).

The modified graft, particularly the bone marrow suspension, may additionally comprise any of stem cells comprised in blood cells, cord blood cells, donor lymphocytes, peripheral blood stem cells, and/or adult stem cells of the bone marrow.

Generally, it is intended that the cell suspension also includes any cell suspension that comprises (any combination of) stem cells, optionally along with any (combination of) other cells.

In preferred embodiments herein, said modified graft is selected from the group consisting of a cell suspension containing T cells and monocytes/macrophages the cell suspension comprising bone marrow cells, non-adherent bone marrow cells, peripheral blood cells, and/or cord blood cells; and a cell suspension comprising lymphocytes, monocytes and/or macrophages.

Further embodiments of the unmodified graft herein correspond to the further particular embodiments described above for the modified graft.

Generally herein, the amount of cells contained in the graft (e.g. in the modified graft) is not particularly limited. Any person skilled in the art will easily be able to choose appropriate amounts of a graft and of cells of the graft for transplantation. Furthermore, suitable guidance is also available e.g. from the specific guidelines for transplantation developed by the "Deutsche Bundesärztekammer", e.g. for human hematopoietic stem cells into patients.

In preferred embodiments herein, said modified graft is a cell suspension and the respective said method (or use, respectively) comprises administration of an amount of from 2×10⁶ cells to 2×10¹⁵ nucleated cells to said subject, wherein preferable amounts will readily be selected by the skilled person. Exemplary preferred ranges include from 1×10⁷ to 1×10¹⁴ nucleated cells, from 1×10⁷ to 1×10¹⁴ nucleated cells, from 1×10⁸ to 1× 10¹³ nucleated cells, from 1×10⁹ to 1×10¹² nucleated cells, from 1×10¹⁰ to 1×10¹¹ nucleated cells, from 1×10⁶ to 1×10⁸ nucleated cells, from 1×10⁷ to 1×10⁹ nucleated cells, from 1×10⁸ to 1×10¹⁰ nucleated cells, from 1×10⁹ to 1×10¹¹ nucleated cells, from 1×10¹⁰ to 1×10¹² nucleated cells, from 1×10¹¹ to 1×10¹³ nucleated cells, from 1×10¹² to 1×10¹⁴ nucleated cells, and from 1×10¹³ to 1×10¹⁵ nucleated cells.

Further embodiments of the unmodified graft herein correspond to the said preferred embodiments described above for the modified graft.

Where the graft to be used herein comprises or is a tissue or an organ, any suitable amounts of said tissue or organ may be administered to said subject. As will be understood by the skilled person, cell numbers in tissues or organs are difficult to determine. Particularly for this reason, the amount of cells contained in the grafts is not particularly limited. Appropriate amounts will easily be determined or selected by the skilled person, e.g. taking into consideration the particular type of subject, graft and/or disease to be treated. In case of organs, the administration of whole organs is preferred.

In a disclosure, the respective said method implies tolerance or partial tolerance within said unmodified graft against the recipient's tissue.

In preferred embodiments of first, second, third and fourth aspects herein, the respective said method i) implies tolerance or partial tolerance within the recipient's tissue against said unmodified graft; and/or ii) implies a reduced likelihood of developing any one of the group consisting of GvHD, donor graft rejection, and organ rejection, upon transplantation of the unmodified graft.

Accordingly, in some disclosure herein, the respective said method implies tolerance or partial tolerance within said unmodified graft against the recipient's tissue. Accordingly, the respective said method may imply tolerance or partial tolerance within the recipient's tissue against said unmodified graft. Accordingly, the respective said method may imply a reduced likelihood of developing any one of the group consisting of GvHD, donor graft rejection, and organ rejection, upon transplantation of the unmodified graft.

The use of the modified graft may imply a reduced likelihood of developing any one of the group consisting of GvHD, donor graft rejection, and organ rejection; particularly of GvHD, upon transplantation of said graft. The use may imply tolerance within the transplanted immunocompetent cells against the recipient's tissue upon transplantation of said modified graft. The use may imply tolerance against the modified graft upon transplantation of said modified graft. The use may imply tolerance or partial tolerance within the recipient's tissue against the modified graft upon transplantation of said modified graft. The use may be for silencing cell activation within said graft. The use may be for reducing the CD4-positive immune cells' ability to be activated within said graft. The use may be for obtaining anergy of the CD4-positive immune cells within said graft. The use may implicate / may be for any combination of the above.

The use of the modified graft may result in a reduced likelihood of developing any one of the group consisting of GvHD, donor graft rejection, and organ rejection; particularly of GvHD, upon transplantation of said graft. The use may result in tolerance of the transplanted immunocompetent cells against the recipient's tissue upon transplantation of said modified graft. The use may result in tolerance against the modified graft upon transplantation of said modified graft. The use may result in tolerance or partial tolerance of the recipient's tissue against the modified graft upon transplantation of said modified graft. The use may be for preventing cell activation or reducing the cells' ability to be activated within said graft. The use may be for reducing the CD4-positive immune cells' ability to be activated within said graft. The use may be for obtaining anergy of the CD4-positive immune cells within said graft. The use may implicate / may be for any combination of the above.

It is disclosed that the said subject (or recipient, respectively) may be allogeneic or xenogeneic with respect to the donor(s).

In the present invention, generally, the said subject (or recipient, respectively) is allogeneic with respect to the donor(s).

As will readily be understood by the skilled person, the recipient and the respective donor(s) are referred to as being allogeneic when they are separate individuals of the same species, whereas the recipient and the respective donor(s) are referred to as being xenogeneic when they are derived from different species.

Accordingly, the said subject is allogeneic with respect to the donor of the said unmodified graft and to the donor of the said modified graft. In other disclosure herein, the said subject is xenogeneic with respect to the donor of the said unmodified graft and to the donor of the said modified graft.

Similarly, in the present invention, generally, the donors may be allogeneic or xenogeneic with respect to each other.

As will readily be understood by the skilled person, the donor(s) are referred to as being allogeneic when they are separate individuals of the same species, whereas the donor(s) are referred to as being xenogeneic when they are derived from different species.

Accordingly, in certain disclosure herein, the donor of the modified graft is xenogeneic with respect to the donor of the said unmodified graft. In certain preferred embodiments herein, the donor of the modified graft is allogeneic with respect to the donor of the unmodified graft.

In the present invention, the nature of and relationship between the donors of the modified graft and the unmodified graft and the recipient are not particularly limited. Hence, in certain non-limiting examples, and e.g. where the donor(s) and recipient are mice, the donor(s) may comprise full HLA mismatches with respect to the recipient. Hence, in certain preferred non-limiting examples, and e.g. where the donor(s) and recipient are mice, the donor(s) may have full HLA mismatches with respect to the recipient.

In other non-limiting examples, the donor(s) may e.g. be haploidentical (such as transplantation between parents and infants) to the recipient and may e.g. be HLA-partially matched family members, parents, siblings or children of the recipient.

In any case, it is preferred in the present invention that the donor(s) and the recipient (or subject, respectively) are HLA-related.

Likewise, in the present invention, the nature of and relationship between the donors of the modified graft and the unmodified graft are not particularly limited. Hence, in certain non-limiting examples, the donor(s) may comprise, preferably have, full HLA mismatches with respect to each other. In other non-limiting examples, one donor may e.g. be haploidentical to the other donor, and may e.g. be HLA-partially matched family members, parents, siblings or children of the other donor.

In any case, it is preferred in the present invention that the modified graft and the unmodified graft are from HLA-related donors. Accordingly, the respective donors in context with the present invention are preferably HLA-related (i.e. HLA-related to each other).

As used herein, the term "HLA-related donors" is preferably understood as referring to donors comprising, preferably having, not more than 50% HLA mismatches. In preferred embodiments, the donors comprise, preferably have, not more than 40% HLA mismatches, preferably not more than 30% HLA mismatches, preferably not more than 20% HLA mismatches, preferably not more than 10% HLA mismatches, preferably no HLA mismatch.

Similarly, a given donor and recipient are referred to as being "HLA-related" when they comprise, preferably have, not more than 50% HLA mismatches. In preferred embodiments, a given donor and recipient comprise, preferably have, not more than 40% HLA mismatches, preferably not more than 30% HLA mismatches, preferably not more than 20% HLA mismatches, preferably not more than 10% HLA mismatches, preferably no HLA mismatch.

Accordingly, and particularly in case of humans, the term "HLA-related" is preferably understood as referring to the presence of not more than 50% HLA mismatches, preferably not more than 40% HLA mismatches, preferably not more than 30% HLA mismatches, preferably not more than 20% HLA mismatches, preferably not more than 10% HLA mismatches, preferably no HLA mismatch.

Accordingly, and particularly in case of humans, the term "HLA-related" is preferably understood as referring to the presence of not more than six HLA mismatches, preferably not more than five HLA mismatches, preferably not more than four HLA mismatches, preferably not more than three HLA mismatches, preferably not more than two HLA mismatches, preferably not more than one HLA mismatch, preferably to no HLA mismatch.

In certain preferred embodiments, in case of humans, the said percentages and or numbers of mismatch(es) are determined with respect to the HLA loci HLA-A, -B, C-, -DR, -DQ, and -DP (corresponding to a total of 12 HLA loci). In other preferred embodiments, the said mismatch(es) are determined with respect to the HLA loci HLA-A, -B, C-, -DR, and - DQ (corresponding to a total of 10 HLA loci). In other preferred embodiments, the said mismatch(es) are determined with respect to the HLA loci HLA-A, -B, C-, and -DR (corresponding to a total of 8 HLA loci).

Generally herein, methods for determining HLA mismatches are not particularly limited, but are readily available and apparent to the person skilled in the art.

Furthermore, as will be readily understood by the skilled person, in the above definitions of the term "HLA related", HLA loci may be replaced by corresponding appropriate loci where non-human donors and recipients are compared to each other. Said loci are readily known to the skilled person.

Moreover, further envisaged herein are cases where the modified graft (e.g. without or after genetic manipulation) is HLA-related to the unmodified graft and vice versa. Hence, in certain preferred embodiments of the present invention, the modified graft and the unmodified graft are "HLA-related", wherein particular embodiments for "HLA-related" correspond to any of those above. Moreover, further envisaged herein are cases where the modified graft and/or the unmodified graft (e.g. without or after genetic manipulation) is HLA-related to the recipient. Hence, in certain preferred embodiments of the present invention, the modified graft is HLA-related to the recipient. Hence, in certain preferred embodiments of the present invention, the unmodified graft is HLA-related to the recipient. Again, particular embodiments for "HLA-related" correspond to any of those above.

Accordingly, as will also be understood by the skilled person, the modified and unmodified grafts may be HLA-related in case the respective donors are not HLA-related - and a given modified graft and/or unmodified may be HLA-related to a recipient in case the respective donor(s) and recipient are not HLA-related. Furthermore, as will also be understood by the skilled person, (grafts or) donors may also be HLA-related in case the donors are xenogeneic - and (modified and unmodified grafts or) a given donor and recipient may also be HLA-related in case the donor and recipient are xenogeneic.

To the latter end, e.g. (one of) the donor(s) (or grafts, respectively) may be genetically manipulated to achieve one or more HLA matches or further HLA matches. Accordingly, in certain preferred embodiments, donor(s) with respect to the recipient (or the donors of the modified graft and the unmodified graft, respectively) preferably comprise, preferably have, not more than six HLA mismatches after genetic manipulation e.g. of donor cells, preferably comprise, more preferably have, not more than three HLA mismatches after genetic manipulation e.g. of donor cells, preferably comprise, more preferably have, not more than two HLA mismatches after genetic manipulation e.g. of donor cells, preferably comprise, more preferably have, not more than one HLA mismatch after genetic manipulation e.g. of donor cells, preferably comprise, more preferably have, no HLA mismatch after genetic manipulation e.g. of donor cells. Further embodiments in the latter context correspond to those as described above.

As also described above, in the present invention, the modified graft and the unmodified graft are preferably from HLA-related donors or, in other words, the donor of the modified graft and the donor of the unmodified graft are preferably HLA-related.

Non-limiting particular examples of HLA-related donors include tissue-type matched donors and related twins. Hence, in certain embodiments, said HLA related donors are tissue-type matched donors (such as a tissue HLA type matched donor). Accordingly, in certain embodiments, the modified graft and the unmodified graft are from tissue-type matched donors. In certain embodiments, said HLA related donors are related twins, especially monozygotic twins. Accordingly, in certain embodiments, the modified graft and the unmodified graft are from related twins, especially from monozygotic twins.

Generally, in particularly preferred embodiments, the modified and unmodified grafts are from the same donor. Accordingly, in preferred embodiments, the said HLA-related donors may be one and the same donor. Hence, the term HLA-related donors as used herein may actually refer to one (particular) donor. Hence, in particular preferred embodiments, said HLA related donors are one particular donor. Hence, in certain preferred embodiments of the present invention, both the modified graft and the unmodified graft are from the same donor.

In preferred embodiments of the invention, the anti-CD4 antibody i) is selected from the group consisting of Max16H5, OKT4A, OKTcdr4a, cMT-412, YHB.46, particularly wherein said anti-CD4 antibody is Max16H5; or ii) is antibody 30F16H5; or iii) is obtainable from a cell line deposited with accession number ECACC 88050502; or iv) is obtainable from a cell line MAX.16H5/30F16H5 deposited with the DSMZ on December 2, 2011; or v) is antibody 16H5.chimIgG4; or vi) is obtainable from a cell line CD4.16H5.chimIgG4 deposited with the DSMZ on December 2, 2011; or vii) is an antibody comprising the VH and the VK of antibody 16H5.chimIgG4; or viii) is an antibody comprising a VH and a VK of an antibody obtainable from a cell line CD4.16H5.chimIgG4 deposited with the DSMZ on December 2, 2011; or ix) is an antibody comprising any combination of a VH selected from SEQ ID NOs: 1-10 and of a VK selected from SEQ ID NOs: 11-20, particularly wherein said combination is selected from VH1/VK1, VH2/VK2, VH4/VK2 and VH4/VK4, especially wherein said combination is VH2/VK2, or x) is a mixture of antibodies selected from the antibodies according to i) to ix) above.

Accordingly, referred anti-CD4 antibodies for use in accordance with the present invention are selected from the group consisting of Max16H5, OKT4A, OKTcdr4a, cMT-412, YHB.46. A particularly preferred anti-CD4 antibody is Max16H5. Cells for the production of Max16H5 have been deposited with the ECACC (European Collection of Cell Cultures) with accession number ECACC 88050502. Said antibody is also disclosed in DE 3919294. As used herein, the antibody "Max16H5" may also be referred to as "Max.16H5", "MAX16H5" or "MAX.16H5", or also "30F16H5" (wherein the latter name is also the name of deposited cells producing said antibody). Max.16H5 may also be obtained from the cell line MAX.16H5/30F16H5 (cf. deposit DSM ACC3148). Another particularly preferred anti-CD4 antibody for use in the invention is 16H5.chimIgG4. As used herein, said antibody may also be referred to as "16H5.chim" or as "CD4.16H5.chimIgG4" (wherein the latter name is also the name of deposited cells producing said antibody). 16H5.chimIgG4 may be obtained from the cell line CD4.16H5.chimIgG4 (cf. deposit DSM ACC3147).

In detail, certain preferred anti-CD4 for use in the present invention are e.g. obtainable from any of the following deposits of biological material: i) deposit with the European Collection of Cell Cultures having the accession number ECACC 88050502 (which is e.g. also described in application DE 3919294); ii) deposit "MAX.16H5/30F16H5", accession number DSM ACC3148, deposited with the DSMZ on December 2, 2011; iii) deposit "CD4.16H5.chimIgG4", accession number DSM ACC3147, deposited with the DSMZ on December 2, 2011. All of these deposits involve cells or cell lines, respectively, from which particular anti-CD4 antibodies in the context with the present invention may be obtained.

The present invention further relates to alternative embodiments of embodiments disclosed herein, where the term "ECACC 88050502" is replaced by "MAX.16H5/30F16H5". Likewise, the present invention further relates to embodiments, where the term "cell line ECACC 88050502" as used herein, or an equivalent term, is replaced by the term "cell line MAX.16H5/30F16H5" or an equivalent term. The present invention further relates to alternative embodiments of embodiments disclosed herein, where the term "ECACC 88050502" is replaced by "CD4.16H5.chimIgG4". Likewise, the present invention further relates to embodiments, where the term "cell line ECACC 88050502" as used herein, or an equivalent term, is replaced by the term "cell line CD4.16H5.chimIgG4" or an equivalent term.

Further, anti-CD4 antibodies (including modified antibodies, etc.) that may be used correspond to the anti-CD4 antibodies described in patent application W02012/072268 (cf. particularly the "additional aspect" described therein). This particularly also applies to the embodiments according to items 1-33 disclosed on pages 42 et seqq. of W02012/072268.

Likewise, methods to obtain / prepare further anti-CD4 antibodies that may be used correspond to those described in W02012/072268 as well as the references disclosed therein. Likewise, methods to evaluate anti-CD4 antibodies (including e.g. method for the measurement of the affinity of antibodies) that may be used in correspond to those described in W02012/072268 as well as the references disclosed therein.

Herein, the term "VH" generally refers to the heavy chain variable region of the heavy chain of an antibody. The "heavy chain variable region" is also referred to as "heavy chain immunoglobulin variable domain". Also these terms are well-known in the art. The term "VL" generally refers to the light chain variable region of the light chain of an antibody. The "light chain variable region" is also referred to as "light chain immunoglobulin variable domain". These terms are well-known in the art, too. VH preferably means a polypeptide that is about 110 to 125 amino acid residues in length. Similarly, VL preferably means a polypeptide that is about 95-130 amino acid residues in length.

In preferred embodiments herein, prior to introduction into said subject, said unmodified graft and/or modified graft is/are additionally incubated with soluble bioactive molecules.

Accordingly, in preferred embodiments herein, prior to introduction into said subject, said unmodified graft is additionally incubated with soluble bioactive molecules. Accordingly, in preferred embodiments herein, prior to introduction into said subject, said modified graft is additionally incubated with soluble bioactive molecules.

In each case, in particular such bioactive molecules are agents promoting immunosuppression, immunotolerance and/or formation of regulatory T cells. Preferably, each case, such bioactive molecules are agents promoting immunosuppression, immunotolerance and/or (favored) formation and/or (favored) activation of regulatory T cells.

Preferred exemplary agents are selected from the group consisting of IL-2, TGF-β, rapamycin, retinoic acid, 4-1BB ligand, and anti-CD28 antibodies; or any combination thereof.

Generally, the grafts for use in the present invention may optionally be administered to the subject together with any medicament or combination of medicaments. This applies to both the modified and unmodified grafts.

Said medicament(s) may be administered prior to, together with and/or following transplantation. Suitable administration modes and routes are not particularly limited and will easily be chosen by the skilled person. Such medicament(s) may support the features or advantages, respectively, of the present uses, modified grafts, or modified grafts for use described hereinabove, such as reducing the likelihood of any one of the group consisting of GvHD, donor graft rejection, and organ rejection. Non-limiting examples for such medicaments include rapamycin and retinoic acid.

The modified graft may be used i) for achieving tolerance or partial tolerance of the modified graft against the recipient's tissue; and/or ii) for achieving tolerance or partial tolerance of the recipient's tissue against the modified graft; and/or iii) for reducing the likelihood of any one of the group consisting of GvHD, donor graft rejection, and organ rejection upon transplantation of said modified graft.

In preferred embodiments herein, the modified graft is used i) for achieving tolerance or partial tolerance of the recipient's tissue against the unmodified graft; and/or ii) for reducing the likelihood of any one of the group consisting of GvHD, donor graft rejection, and organ rejection upon transplantation of said unmodified graft.

Disclosed are methods of treating a subject in need of such treatment in accordance with the used described herein. Said grafts, subjects, methods, and/or diseases may be as described hereinabove.

Disclosed is an unmodified graft, a modified graft and/or an anti-CD4 antibody for use in the manufacture of a medicament for the treatment of one or more diseases treatable by transplantation in a subject and/or for any of the (medical) uses described herein. Said use may be further defined as described herein above. Accordingly, said grafts, subjects, methods, and/or diseases may be as described hereinabove.

The graft (particularly the modified graft) for use in the present invention, may or may not comprise stem cells. That is, the cell graft containing immune cells may be replaced by any graft and includes a graft comprising stem cells as well as a graft not comprising stem cells. In other words, the graft for use may be any cell graft containing immune cells. In even other words, in certain embodiments a graft used in accordance with the invention comprises stem cells, whereas in other embodiments, a graft used in accordance with the invention does not comprise stem cells. In certain embodiments, the graft does not comprise isolated CD4⁺ cells. In certain embodiments, the graft does not comprise purified D0.11.10 CD4⁺ T cells.

Generally, further embodiments of the various aspects herein correspond to those described in W02012/072268.

Generally, as to further mechanistic, experimental and theoretical characteristics related to (side) aspects of the present invention, reference is also made to W02012/072268.

Generally, the invention also relates to embodiments, where the term "comprises" or an equivalent term is replaced by "has" or an equivalent term. For example, the invention generally also relates to embodiments, where the term "comprising" or an equivalent term is replaced by "having" or an equivalent term.

In the following, the present invention is further illustrated by examples which are not intended to limit the scope of the present invention.

### EXAMPLES

### Example 1:

### Animals and experimental design

Donor triple transgenic mice (human CD4^{+/+}, murine CD4^{-/-}, HLA-DR3^{+/+}) TTG-C57Bl/6 were bred at the Animal Facility at the University Leipzig [Fricke, 2014; Schmidt, 2015]. Recipient Balb/c^{wt} mice were purchased from Charles River (Sulzfeld, Germany; http://jaxmice.jax.org) [Fricke, 2014; Schmidt, 2015]. All mice were maintained under standardized conditions [Fricke, 2014; Schmidt, 2015]. In transgenic mice, the CD4 transgene includes its own promoter ligated to a murine CD4 enhancer element thus leading to T cell subset-specific expression [Fricke, 2014; Schmidt, 2015]. Murine CD8⁺ cells are not affected [Fricke, 2014; Schmidt, 2015]. Transgenic mice express the HLA-DR3 molecule in addition to the murine MHC II complex [Fricke, 2014; Schmidt, 2015]. The TTG-C57Bl/6 mice have complete functional murine immune systems [Fricke, 2014; Schmidt, 2015]. The mice were fed *ad libitum* [Fricke, 2014; Schmidt, 2015]. All mice were housed, treated, or handled in accordance with the guidelines of the University Leipzig Animal Care Committee and were approved by the Regional Board of Animal Care for Leipzig [Fricke, 2014; Schmidt, 2015].

Allogeneic hematopoietic stem cell grafts from donor TTG-C57BI/6 were incubated with MAX.16H5 IgG₁ or control antibodies for 2 hours before transplantation into Balb/c^{wt} [Fricke, 2014; Schmidt, 2015]. Survival and GvHD occurrence were measured daily, engraftment, hematological (WBC subsets) and immunological reconstitution (murine CD4, human CD4, murine CD8, HLA-DR3, and H2K^{b}) were measured weekly [Fricke, 2014; Schmidt, 2015].

### Irradiation protocol

For the irradiation of mice, the X-Ray apparatus (D3225, Orthovoltage, Gulmay Medical, Camberley, UK) was adjusted for animal irradiation as we described previously [Fricke, 2009]. Irradiation was performed before transplantation of recipient mice.

### Preparation of bone marrow cells, splenocytes, and antibody incubation

As isotype control, the antibody from LEAF^{™} Purified Mouse IgG₁ (κ, Isotype Ctrl Antibody, San Diego, CA 92121) was used [Fricke, 2014]. MAX.16H5 IgG₁ antibodies were used as described previously [Fricke, 2014]. Bone marrow cells (BM) and splenocytes were prepared as described elsewhere [Fricke, 2009; Fricke, 2010; Fricke, 2011; Fricke, 2012, Fricke, 2014, Schmidt, 2015]. For antibody incubation, the calculated amount of antibodies was diluted before use to a final concentration of 1mg/ml in DMEM without FCS [Fricke, 2014]. Subsequently, 1.4×10⁸ of BM and 1.4×10⁸ of splenocytes from donors were incubated with 800µg MAX.16H5 IgG₁ in 15ml DMEM without FCS at room temperature in the dark for 2 hours [Fricke, 2014]. As control, BM and splenocytes from the donors without antibody pre-incubation were prepared under the same conditions [Fricke, 2014]. After 2 hours of incubation, cells were centrifuged at 300g for 10 min to pellet them and washed once in PBS (1x) at 300g for 10 min to remove unbound antibodies [Fricke, 2014].

### Cell Transplantation

For co-transplantation experiments, 2×10⁷ pre-incubated bone marrow cells of donor TTG-C57Bl/6 mice were added to 2×10⁷ MAX.16H5 IgG₁ pre-incubated splenocytes [Fricke, 2014]. The cell concentration was adjusted to a final volume of 150µl sterile 0.9% NaCl [Fricke, 2014]. Subsequently, the grafts were allogeneically transplanted by intravenous injection into the lateral tail vein of lethally irradiated recipient Balb/c^{wt} mice [Fricke, 2014]. Survival, GvHD symptoms, and weight were assessed every day after transplantation [Fricke, 2014].

### Flow cytometry

Before and after transplantation, donor TTG-C57Bl/6 and recipient Balb/c^{wt} mice were analyzed by flow cytometry. For cytometric analysis, cells were prepared and incubated as previously described [Fricke, 2009; Fricke, 2010; Fricke, 2011; Fricke, 2012, Fricke, 2014]. Additionally, the viability of splenocytes and bone marrow cells was tested before transplantation by staining with 7-Amino-Actinomycin D (7-AAD). 1×10⁶ cells were incubated with 5µl (0.25µg/test) of 7-AAD in 300µl PBS at room temperature for 30 min and measured immediately. Before and after transplantation, recipient Balb/c^{wt} mice were analyzed by flow cytometry and the full blood cell counts were determined. The following antibodies were used: murine CD4-PECy7, MHC-I (H-2D[b]-PE, and murine CD8-PerCP [BD Biosciences, Heidelberg, Germany]; murine CD3-FITC and human CD4-APC [Beckman Coulter, Krefeld, Germany]; and human HLA-DR3-FITC [Immunotools, Friesoythe, Germany]. For cytometric analysis of murine FoxP3, the murine T_{reg} Detection Kit (Miltenyi Biotec GmbH, Bergisch Gladbach, Germany) was used according to the manufacturer's protocol. The data were acquired on a BD FACSCanto^{™} II Flow Cytometer and analyzed using BD FACSDiva^{™} software (both BD Biosciences, Heidelberg, Germany) [Fricke, 2014].

### Skin transplantation

For allogeneic skin transplantation Balb/c^{wt} (TTG) mice (showing C57Bl/6 blood chimerism) and Balb/c^{wt} mice were used as recipient and CD4/CD3 mice (TTG) as donors. Recipient mice were anesthetized by a standard protocol approved by the authorities. Skin of the donor mice from the tails was immediately prepared. The fur on the shoulder of recipient mice was shaved carefully before transplantation. Full-thickness grafts of 5x5mm donor tail skin (TTG) were transplanted on Balb/c (TTG) or Balb/c^{wt} recipients. Transplanted skin graft was secured by surgical sterile thread and additionally dressed with adhesive sterile bandage. Recipient mice were awakened by a standard protocol approved by the authorities. After surgery, mice were individually housed to avoid additional injuries. All procedures of skin transplantation took place under sterile conditions in an operating room and were approved by the authorities. Mice were monitored daily. Fifty days (50 days) after skin transplantation, the tissue was collected for histological analysis.

### Histology of recipient mice

Histology was done as described previously [Fricke, 2011].

### Statistical Analysis

All data are presented as means ± standard deviation [Fricke, 2012]. Statistical analysis and graphic presentations were made using SigmaPlot 10.0/SigmaStat 3.5 software (SYSTAT, Erkrath, Germany) [Fricke, 2012] and GraphPad Prism 5 (v5.03, GraphPad Software Inc, Dan Diego, California). P-values calculated with SigmaPlot 11.0/SigmaStat 3.5 software. Test variables were the GvHD score, weight, survival, and cell numbers. Analysis of survival curves was done using the log-rank test, analysis of other parameters with t-tests, the Mann-Whitney U test or the Holm-Sidak test [Fricke, 2009].

### Results: (cf. also Fig. 1)

It was hypothesized that solid organs (e.g. skin) from third party TTG donor mice on Balb/c (TTG) recipient mice, which showed immune tolerance, would not be rejected after transplantation. For this, 5 Balb/c (TTG) mice (showing C57Bl/6 blood chimerism) and 4 Balb/c^{wt} (control) were transplanted using tail skin of TTG mice. It had to be mentioned that Balb/c (TTG) recipients were about 6 months older as control mice because of hematopoietic stem cell transplantation before. Sixty percent (60%) of skin transplanted Balb/c (TTG) mice survived and 100% of Balb/c^{wt} mice. Nevertheless, all surviving Balb/c (TTG) mice (showing C57Bl/6 blood chimerism, N=3), which received a third party skin graft from TTG mice did not show graft rejection unlike the controls (Fig. 1). Graft failure of TTG skin was observed in the Balb/c^{wt} control group (N=4).

### Example 2:

As to further antibodies for use in the present invention, chimeric anti-CD4 antibodies may be made, and modified anti-CD4 antibodies may be designed and generated in accordance with Example 2 of W02012/072268.

### Example 3:

Furthermore, as to an animal model for the human immune system that may supplementarily be used by the skilled person in context with certain issues of this invention, reference is made to WO 2006/122545 including all family members of the respective patent family such as EP 1887859 and US 2008216182.

### LIST OF REFERENCES

DE 3919294
WO 2006/122545
WO 2012/072268
Barnard, C.N. The operation. A human cardiac transplant: an interim report of a successful operation performed at Groote Schuur Hospital, Cape Town. S Afr Med J. 41, 1271-1274 (1967).
Becker, C., Bopp, T. & Jonuleit,H. Boosting regulatory T cell function by CD4 stimulation enters the clinic. Front Immunol. 3. 164 (2012).
Bellone, M. Autoimmune Disease: Pathogenesis. Encyclopedia of Life Sciences John Wiley & Sons, 1-8 (2005).
Bowers, K., Pitcher, C. & Marsh, J.M. CD4:A co-receptor in the immune response and HIV infection.. Int. J. Biochem. Cell Biol.. 29, 871-875 (1997).
Calne, R.Y. et al. A study of the effects of drugs in prolonging survival of homologous renal transplants in dogs. Ann N Acad Sci. 99, 743-761 (1962).
Carrel, A. & Guthrie, C.C. Successful Transplantation of Both Kidneys from a Dog into a Bitch with Removal of Both Normal Kidney from the Latter. Science 23, 394-395 (1906).
Chatenoud, L., Waldmann, H., & Emmrich,F. Tolerance induction in the adult: 'danger at Le Bischenberg. Immunol. Today 16, 121-123 (1995).
Coligan et al., Current Protocols in Immunology, John Wiley & Sons 1991-1997
Cooke KR, Kobzik L, Martin TR, Brewer J, Delmonte J, Crawford JM et al. An experimental model of idiopathic pneumonia syndrome after bone marrow transplantation .1. The roles of minor H antigens and endotoxin. Blood 88(8):3230-3239 (1996)
Cooke, K.R. et al. Tumor necrosis factor- alpha production to lipopolysaccharide stimulation by donor cells predicts the severity of experimental acute graft-versus-host disease. J. C/in. Invest 102, 1882-1891 (1998).
Cooley, D.A. et al. Organ transplantation for advanced cardiopulmonary disease. Ann Thorac Surg. 8, 30-46 (1969).
Dausset, J. Iso-leuco-anticorps. Acta Haematol. 20, 1956-1966 (1958).
De Castello, A. & Sturli, A. Ueber die Isoagglutinine im Serum gesunder und kranker Menschen. Mfinch med Wschr. 49, 1090-1095 (1902).
De Groot, A.S. & Scott, D.W. Immunogenicity of protein therapeutics. Trend Immunol. 28, 482-490 (2007).
Durand, J.K. & Willis, M.S. Karl Landsteiner, MD: Transfusion Medicine. Laboratory Medicine 41, 53-55 (2009).
Eigler, F.W. Zur Geschichte der Nierentransplantation in Deutschland. Zentralbl Chir. 127, 1001-1008 (2002).
Emmrich, F. et al. An anti-CD4 antibody for treatment of chronic inflammatory arthritis. Agents Actions Suppl 32, 165-170 (1991*a).*
Emmrich, J., Seyfarth, M., Fleig, W.E., & Emmrich, F. Treatment of inflammatory bowel disease with anti-CD4 monoclonal antibody. Lancet 338, 570-571 (1991b).
Eurotransplant. Total numbers of organ transplantations in Germany in the period from 1963 to 2013 according to organs. 2015a [cited on June 19, 2015, URL: http://de.statista.com/statistik/daten/studie/202037/umfrage/anzahl-der-organtransplantationen-in-deutschland/ ]
Eurotransplant. 2015b [cited on June 19, 2015, URL: https://www.eurotransplant. org/cms/index.php?page=pat_germany]
Fricke, S. et al. Allogeneic non-adherent bone marrow cells facilitate hematopoietic recovery but do not lead to allogeneic engraftment. PLoS. One. 4. e6157 (2009).
Fricke, S. et al. Characterization of murine non-adherent bone marrow cells leading to recovery of endogenous hematopoiesis. Cell Mol. Life Sd. 67, 4095-4106 (2010).
Fricke, S. Measurement and illustration of immune interaction after stem cell transplantation. Methods Mol. Biol. 690. 315-332 (2011).
Fricke, S. et al. Allogeneic bone marrow grafts with high levels of CD4(+) CD25(+) FoxP3(+) T cells can lead to engraftment failure. Cytometry A 81, 476-488 (2012).
Fricke, S. et al. Prevention of graft-versus-host-disease with preserved graft-versus-leukemia-effect by ex vivo and in vivo modulation of CD4+ T-cells. Cell Mol Life Sci. 71, 2135-2148 (2014).
Harding, S., Lipp, P., & Alexander, D.R. A therapeutic CD4 monoclonal antibody inhibits TCR-zeta chain phosphorylation, zeta-associated protein of 70-kDa Tyr31 9 phosphorylation, and TCR internalization in primary human T cells. J. Immunol. 169, 230-238 (2002).
Harrison, S.C. CD4: Structure and Interactions of an Immunoglobulin Superfamily Adhesion Molecule. Acc Chem. Res. 26, 449-453 (1993).
Hsu, D.C. & Katelaris, C.H. Long-term management of patients taking immunosuppressive drugs. Australian Prescriber 22, 68-71 (2009).
Jiang, S. & Dong, C. A Complex issue on CD4+ T-cell subsets. Immunol Rev. 252, 5-11 (2013).
June, C.H. et al. Inhibition of tyrosine phosphorylation prevents T-cell receptor-mediated signal transduction. Proc.Natl.Acad.Sci. 87, 7722-7726 (1990).
Kapturczak, M.H. et al. Pharmacology of calcineurin antagonists. Transplant Proc. 36, 25-32 (2004).
Kelly, W.D. et al. Allotransplantation of the pancreas and duodenum along with the kidney in diabetic nephropathy. Surgery 61, 827-837 (1967).
Landsteiner, K. Ueber Agglutinationserscheinungen normalen menschlichen Blutes. Wiener Klinische Wochenschrift 49, 1132-1133 (1901).
Laub R. et al. A multiple transgenic mouse model with a partially humanized activation pathway for helper T cell responses. J. Immunol. Methods 246, 37-50 (2000).
Laub, R., Dorsch, M., Wenk, K., & Emmrich, F. Induction of immunologic tolerance to tetanus toxoid by anti-human CD4 in HLA-DR3(+)/human CD4(+)/murine CD4(-) multiple transgenic mice. Transplant. Proc. 33, 2182-2183 (2001).
Laub, R. et al. Anti-human CD4 induces peripheral tolerance in a human CD4+, murine CD4-, HLA-DR+ advanced transgenic mouse model. J. Immunol. 169, 2947-2955 (2002).
Liu,W. et al. CD127 expression inversely correlates with FoxP3 and suppressive function of human CD4+ T reg cells. J. Exp. Med. 203, 1701-1711 (2006).
Luckheeram, R.V. et al. CD4+ T cells: differentiation and functions. Clin Dev Immunol. 2012, 1-12 (2012).
Madrenas, J., Schwartz, R.H., & Germain,R.N. Interleukin 2 production, not the pattern of early T-cell antigen receptor-dependent tyrosine phosphorylation, controls anergy induction by both agonists and partial agonists. Proc. Nat!. Acad. Sci. U. S. A 93, 9736-9741 (1996).
Madrenas, J. & Germain, R.N. Variant TOR ligands: new insights into the molecular basis of antigen-dependent signal transduction and T-cell activation. Semin. Immunol. 8, 83- 101 (1996).
Medawar, P.B. The behaviour and fate of skin autografts and skin homografts in rabbits. J Anat. 78, 176-199 (1944).
Murray, J.E. et al. Renal transplantation: a twenty-five year experience. Ann Surg. 184, 565-573 (1976).
O'Connell, R.M. et al. MicroRNA-155 promotes autoimmune inflammation by enhancing inflammatory T cell development. Immunity. 33, 607-619 (2010).
Pfitzmann R, Neuhau P, Hetzer R (Hrsg.): Organtransplantation, ISBN 3-11-016849-9
Reinke, P. et al. Anti-CD4 therapy of acute rejection in long-term renal allograft recipients. Lancet 338. 702-703 (1991).
Reinke, P. et al. Late acute rejection in long-term renal allograft recipients. Diagnostic and predictive value of circulating activated T cells. Transplantation 58, 35-41 (1994).
Reinke, P. et al. Anti-CD4 monoclonal antibody therapy of late acute rejection in renal allograft recipients--CD4+ T cells play an essential role in the rejection process. Transplant. Proc. 27, 859-862 (1995).
Robey, E. & Axel, R. CD4:Collaborator in immune recognition and HIV infection. Cell 60, 697-700 (1990).
Schmidt F. et al., Flow cytometric analysis of the graft-versus-Leukemia-effect after hematopoietic stem cell transplantation in mice. Cytometry A. 87(4): 334-45 (2015).
Stewart, S.J. et al. Human T lymphocytes and monocytes bear the same Leu-3(T4) antigen. J Immunol. 136, 3773-3778 (1986).
Sayegh, M.H. et al. Transplantation 50 Years Later-Progress, Challenges, and Promises. N Engl J Med. 351, 2761-2766 (2004).
Schulz, K.-H. & Koch, U. Transplantationspsychiologie. In Balck, F. Anwendungsfelder der medizinischen Psychiologie. Springer, 101-114 (2005).
Starzl, T.E. et al. Orthotopic homotransplantation of the human liver. Ann Surg. 168, 392-415 (1968).
Starzl, T.E. et al. Evolution of liver transplantation. Hepatology 2, 614-636 (1982).
Strauss, G., Vignali.D.A.. Schonrich,G.. & Hammerling,G.J. Negative and positive selection by HLA-DR3(DRw17) molecules in transgenic mice. Immunogenetics 40. 104-108 (1994).

## Claims

1. An unmodified graft for use in a method of treating one or more disease(s) treatable by transplantation in a subject,
wherein said method comprises a first step of introducing into said subject a modified graft, and a second step of introducing into said subject said unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
a) incubating a cell suspension graft containing immune cells with the anti-CD4 antibody, wherein said incubating is carried out for from 1 minute to 7 days, and
b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

2. A modified graft for use in a method of treating one or more disease(s) treatable by transplantation,
wherein said method comprises a first step of introducing into said subject said modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
a) incubating a cell suspension graft containing immune cells with the anti-CD4 antibody, wherein said incubating is carried out for from 1 minute to 7 days, and
b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

3. An anti-CD4 antibody, for use in a method of treating one or more disease(s) treatable by transplantation;
wherein said method comprises a first step of introducing into said subject a modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
a) incubating a cell suspension graft containing immune cells with the anti-CD4 antibody, wherein said incubating is carried out for from 1 minute to 7 days, and
b) removing unbound anti-CD4 antibody from said graft, thereby reducing the amount of unbound antibody in said graft,
and wherein said unmodified graft and said modified graft are allogeneic grafts and comprise immune cells bearing the CD4 antigen.

4. An unmodified graft, a modified graft and/or an anti-CD4 antibody, for use in
i) a surgical transplantation method,
ii) a method of inducing transplantation tolerance, wherein a modified graft is used for inducing transplantation tolerance towards an unmodified graft,
iii) a method of transferring one or more cell suspension(s), particularly stem cell containing cell suspension(s) from a donor to a subject,
iv) a method of transferring one or more tissue(s) from a donor to a subject,
v) a method of transferring one or more partial organ(s) from a donor to a subject,
vi) a method of transferring one or more organ(s) from a donor to a subject,
vii) a method of transferring hematopoietic system reconstituting cells from a donor source into a subject, optionally followed by transferring one or more organ(s), from said donor source into said subject,
or
viii) a method for enhancing immune reconstitution in a subject, optionally followed by transferring one or more organ(s) into said subject;
wherein said method comprises a first step of introducing into said subject a modified graft, and a second step of introducing into said subject an unmodified graft,
wherein said modified graft is a cell suspension graft containing immune cells, that comprises an anti-CD4 antibody, bound to CD4 epitopes of said cell suspension graft, and wherein said modified graft is obtainable by an *in vitro* method comprising the following steps:
a) incubating a cell suspension graft containing immune cells with the anti-CD4 antibody, wherein said incubating is carried out for from 1 minute to 7 days, and
b) removing unbound anti-CD4 antibody from said graft,
and wherein said unmodified graft and said modified graft are allogeneic grafts and comprise immune cells bearing the CD4 antigen.

5. The unmodified graft for use, modified graft for use, or anti-CD4 antibody for use of any of the preceding claims,
wherein said first step is carried out from 30 min to 50 years, particularly from one hour to two months before said second step.
or
wherein said first step is carried out at least one hour, particularly at least three weeks before said second step.

6. The unmodified graft for use, modified graft for use, or anti-CD4 antibody for use of any of the preceding claims, wherein said modified graft comprises stem cells.

7. The unmodified graft for use, modified graft for use, or anti-CD4 antibody for use of any of the preceding claims,
i) wherein said unmodified graft is a cell suspension,
or
ii) wherein said unmodified graft is a tissue,
or
iii) wherein said unmodified graft is an organ.

8. The unmodified graft for use, modified graft for use, or anti-CD4 antibody for use of any of the preceding claims, wherein said modified graft is a cell suspension graft containing immune cells that comprises an anti-CD4 antibody, bound to from 40 % to 100 % of the CD4 epitopes thereof.

9. The unmodified graft for use, modified graft for use, or anti-CD4 antibody for use of claim 8;
wherein said incubating in step a) of said *in vitro* method is carried out
i) for from 1 to 150 minutes, particularly for from 5 minutes to 150 minutes, more particularly for from 10 minutes to 150 minutes, more particularly for from 30 minutes to 150 minutes, more particularly for from 40 minutes to 120 minutes, more particularly for from 45 minutes to 90 minutes, especially for from 50 minutes to 70 minutes;
or
ii) for from 150 minutes to 7 days, particularly for from 150 minutes to 5 days, more particularly from 150 minutes to 3 days, more particularly from 150 minutes to 1 day, especially for from 150 minutes to 8 hours;
and/or
wherein said incubating in step a) of said *in vitro* method is carried out with an antibody amount of from 0.1 µg/ml to 10 mg/ml,
and/or
wherein said incubating in step a) of said *in vitro* method is carried out with an antibody amount of from 0.1pg/1 × 10⁹ nucleated cells to 100 mg/2 × 10⁶ nucleated cells,
and/or
wherein said removing unbound antibody from said graft in step b) is carried out by washing the modified graft at least one time.

10. The unmodified graft for use, modified graft for use, or anti-CD4 antibody for use of any of the preceding claims, wherein said method
i) implies tolerance or partial tolerance within the recipient's tissue against said unmodified graft;
and/or
ii) implies a reduced likelihood of developing any one of the group consisting of GvHD, donor graft rejection, and organ rejection, upon transplantation of the unmodified graft.

11. The unmodified graft for use, modified graft for use, or anti-CD4 antibody for use of any of the preceding claims,
i) wherein said modified graft is selected from the group consisting of a cell suspension containing T cells and monocytes/macrophages the cell suspension comprising bone marrow cells, non-adherent bone marrow cells, peripheral blood cells, and/or cord blood cells; and a cell suspension comprising lymphocytes, monocytes and/or macrophages;;
and/or
ii) wherein said method comprises administration of an amount of from 2 × 10⁶ cells to 2 × 10¹⁵ nucleated cells to said subject.

12. The unmodified graft for use, modified graft for use, or anti-CD4 antibody for use of any of the preceding claims, wherein
A) the modified graft and the unmodified graft are from HLA-related donors; particularly
i) wherein the said HLA-related donors comprise no more than a total of 50% of HLA mismatches, preferably not more than 40% HLA mismatches, preferably not more than 30% HLA mismatches, preferably not more than 20% HLA mismatches, preferably not more than 10% HLA mismatches, preferably no HLA mismatch;
and/or
ii) wherein said HLA-related donors are selected from the group consisting of tissue-type matched donors, related twins or are the same donor;
especially wherein both the modified graft and the unmodified graft are from the same donor,
and/or
B) wherein said modified graft and the unmodified graft are HLA-related; particularly wherein said HLA-related grafts comprise no more than a total of 50% of HLA mismatches, preferably not more than 40% HLA mismatches, preferably not more than 30% HLA mismatches, preferably not more than 20% HLA mismatches, preferably not more than 10% HLA mismatches, preferably no HLA mismatch.

13. The unmodified graft for use, modified graft for use, or anti-CD4 antibody for use of any of claims 3 to 12, wherein said anti-CD4 antibody
i) is selected from the group consisting of Max16H5, OKT4A, OKTcdr4a, cMT-412, YHB.46, particularly wherein said anti-CD4 antibody is Max 16H5;
or
ii) is antibody 30F16H5;
or
iii) is obtainable from a cell line deposited with accession number ECACC 88050502;
or
iv) is obtainable from a cell line MAX.16H5/30F16H5 deposited with the DSMZ on December 2, 2011;
or
v) is antibody 16H5.chimIgG4;
or
vi) is obtainable from a cell line CD4.16H5.chimIgG4 deposited with the DSMZ on December 2, 2011;
or
vii) is an antibody comprising the VH and the VK of antibody 16H5.chimIgG4; or
viii) is an antibody comprising a VH and a VK of an antibody obtainable from a cell line CD4.16H5.chimIgG4 deposited with the DSMZ on December 2, 2011;
or
ix) is an antibody comprising any combination of a VH selected from SEQ ID NOs: 1-10 and of a VK selected from SEQ ID NOs: 11-20, particularly wherein said combination is selected from VH1/VK1, VH2/VK2, VH4/VK2 and VH4/VK4, especially wherein said combination is VH2/VK2,
or
x) is a mixture of antibodies selected from the antibodies according to i) to ix) above.

14. The unmodified graft for use, modified graft for use, or anti-CD4 antibody for use of any of the preceding claims,
wherein, prior to introduction into said subject, said unmodified graft and/or modified graft is/are additionally incubated with agents promoting immunosuppression, immunotolerance and/or formation of regulatory T cells,
especially with an agent selected from the group consisting of Il-2, TGF-β, rapamycin, retinoic acid, 4-1BB ligand, and anti-CD28 antibodies; or any combination thereof.

15. The unmodified graft for use, modified graft for use, or anti-CD4 antibody for use of any of the preceding claims, wherein the modified graft is used
i) for achieving tolerance or partial tolerance within the recipient's tissue against the unmodified graft;
and/or
ii) for reducing the likelihood of any one of the group consisting of GvHD, donor graft rejection, and organ rejection, upon transplantation of said unmodified graft.

## Patentansprüche

1. Unmodifiziertes Transplantat zur Verwendung in einem Verfahren zur Behandlung einer oder mehrerer Krankheiten, die durch Transplantation bei einem Subjekt behandelt werden können,
wobei das Verfahren einen ersten Schritt des Einbringens eines modifizierten Transplantats in das Subjekt und einen zweiten Schritt des Einbringens des unmodifizierten Transplantats in das Subjekt umfasst,
wobei das modifizierte Transplantat ein Immunzellen enthaltendes ZellsuspensionsTransplantat ist, das einen Anti-CD4-Antikörper umfasst, der an CD4-Epitope des Zellsuspensions-Transplantats gebunden ist, und wobei das modifizierte Transplantat durch ein In-vitro-Verfahren erhältlich ist, das die folgenden Schritte umfasst:
a) Inkubieren eines Immunzellen enthaltenden Zellsuspensions-Transplantats mit dem Anti-CD4-Antikörper, wobei das Inkubieren für von 1 Minute bis 7 Tage durchgeführt wird, und
b) Entfernen von ungebundenem Anti-CD4-Antikörper von dem Transplantat, wodurch die Menge des ungebundenen Antikörpers in dem Transplantat verringert wird,
und wobei das unmodifizierte Transplantat und das modifizierte Transplantat allogene Transplantate sind und Immunzellen umfassen, die das CD4-Antigen tragen.

2. Modifiziertes Transplantat zur Verwendung in einem Verfahren zum Behandeln einer oder mehrerer Krankheiten, die durch Transplantation behandelt werden können,
wobei das Verfahren einen ersten Schritt des Einbringens des modifizierten Transplantats in das Subjekt und einen zweiten Schritt des Einbringens eines unmodifizierten Transplantats in das Subjekt umfasst,
wobei das modifizierte Transplantat ein Immunzellen enthaltendes ZellsuspensionsTransplantat ist, das einen Anti-CD4-Antikörper umfasst, der an CD4-Epitope des Zellsuspensions-Transplantats gebunden ist, und wobei das modifizierte Transplantat durch ein In-vitro-Verfahren erhältlich ist, das die folgenden Schritte umfasst:
a) Inkubieren eines Immunzellen enthaltenden Zellsuspensions-Transplantats mit dem Anti-CD4-Antikörper, wobei das Inkubieren für von 1 Minute bis 7 Tage durchgeführt wird, und
b) Entfernen von ungebundenem Anti-CD4-Antikörper von dem Transplantat, wodurch die Menge des ungebundenen Antikörpers in dem Transplantat verringert wird,
und wobei das unmodifizierte Transplantat und das modifizierte Transplantat allogene Transplantate sind und Immunzellen umfassen, die das CD4-Antigen tragen.

3. Anti-CD4-Antikörper zur Verwendung in einem Verfahren zum Behandeln einer oder mehrerer durch Transplantation behandelbarer Krankheit(en);
wobei das Verfahren einen ersten Schritt des Einbringens eines modifizierten Transplantats in das Subjekt und einen zweiten Schritt des Einbringens eines unmodifizierten Transplantats in das Subjekt umfasst,
wobei das modifizierte Transplantat ein Immunzellen enthaltendes ZellsuspensionsTransplantat ist, das einen Anti-CD4-Antikörper umfasst, der an CD4-Epitope des Zellsuspensions-Transplantats gebunden ist, und wobei das modifizierte Transplantat durch ein In-vitro-Verfahren erhältlich ist, das die folgenden Schritte umfasst:
a) Inkubieren eines Immunzellen enthaltenden Zellsuspensions-Transplantats mit dem Anti-CD4-Antikörper, wobei das Inkubieren für von 1 Minute bis 7 Tage durchgeführt wird, und
b) Entfernen von ungebundenem Anti-CD4-Antikörper von dem Transplantat, wodurch die Menge des ungebundenen Antikörpers in dem Transplantat verringert wird,
und wobei das unmodifizierte Transplantat und das modifizierte Transplantat allogene Transplantate sind und Immunzellen umfassen, die das CD4-Antigen tragen.

4. Unmodifiziertes Transplantat, modifiziertes Transplantat und/oder ein Anti-CD4-Antikörper, zur Verwendung in
i) einem chirurgischen Transplantationsverfahren,
ii) einem Verfahren zum Induzieren von Transplantationstoleranz, wobei ein modifiziertes Transplantat zum Induzieren von Transplantationstoleranz gegenüber einem nicht modifizierten Transplantat verwendet wird,
iii) einem Verfahren zum Transferieren einer oder mehrerer Zellsuspension(en), insbesondere stammzell-enthaltenden Zellsuspension(en), von einem Donor auf ein Subjekt,
iv) einem Verfahren zum Transferieren von einem oder mehreren Geweben von einem Donor auf ein Subjekt,
v) einem Verfahren zum Transferieren eines oder mehrerer Teilorgane von einem Donor auf ein Subjekt,
vi) einem Verfahren zum Transferieren eines oder mehrerer Organe von einem Donor auf ein Subjekt,
vii) einem Verfahren zum Transferieren von Zellen zum Wiederherstellen des hämatopoetischen Systems von einer Donorquelle in ein Subjekt, gegebenenfalls gefolgt vom Transferieren eines oder mehrerer Organe von der Donorquelle in das Subjekt,
oder
viii) einem Verfahren zur Verstärkung der Immunrekonstitution in einem Subjekt, gegebenenfalls gefolgt vom Transferieren eines oder mehrerer Organe in da Subjekt;
wobei das Verfahren einen ersten Schritt des Einbringens eines modifizierten Transplantats in das Subjekt und einen zweiten Schritt des Einbringens eines unmodifizierten Transplantats in das Subjekt umfasst,
wobei das modifizierte Transplantat ein Immunzellen enthaltendes ZellsuspensionsTransplantat ist, das einen Anti-CD4-Antikörper umfasst, der an CD4-Epitope des Zellsuspensions-Transplantats gebunden ist, und wobei das modifizierte Transplantat durch ein In-vitro-Verfahren erhältlich ist, das die folgenden Schritte umfasst:
a) Inkubieren eines Immunzellen enthaltenden Zellsuspensions-Transplantats mit dem Anti-CD4-Antikörper, wobei das Inkubieren für von 1 Minute bis 7 Tage durchgeführt wird, und
b) Entfernen von ungebundenem Anti-CD4-Antikörper von dem Transplantat, wodurch die Menge des ungebundenen Antikörpers in dem Transplantat verringert wird,
und wobei das unmodifizierte Transplantat und das modifizierte Transplantat allogene Transplantate sind und Immunzellen umfassen, die das CD4-Antigen tragen.

5. Unmodifiziertes Transplantat zur Verwendung, modifiziertes Transplantat zur Verwendung oder Anti-CD4-Antikörper zur Verwendung nach einem beliebigen der vorangegangenen Ansprüche,
wobei der erste Schritt von zwischen 30 Minuten und 50 Jahre, insbesondere von zwischen einer Stunde und zwei Monate vor dem zweiten Schritt durchgeführt wird. oder
wobei der erste Schritt mindestens eine Stunde, insbesondere mindestens drei Wochen vor dem zweiten Schritt durchgeführt wird.

6. Unmodifiziertes Transplantat zur Verwendung, modifiziertes Transplantat zur Verwendung oder Anti-CD4-Antikörper zur Verwendung nach einem beliebigen der vorangegangenen Ansprüche, wobei das modifizierte Transplantat Stammzellen umfasst.

7. Unmodifiziertes Transplantat zur Verwendung, modifiziertes Transplantat zur Verwendung oder Anti-CD4-Antikörper zur Verwendung nach einem beliebigen der vorangegangenen Ansprüche,
i) wobei das unmodifizierte Transplantat eine Zellsuspension ist,
oder
ii) wobei das unmodifizierte Transplantat ein Gewebe ist,
oder
iii) wobei das unmodifizierte Transplantat ein Organ ist.

8. Unmodifiziertes Transplantat zur Verwendung, modifiziertes Transplantat zur Verwendung oder Anti-CD4-Antikörper zur Verwendung nach einem beliebigen der vorangegangenen Ansprüche, wobei das modifizierte Transplantat ein Immunzellen enthaltendes Zellsuspensionstransplantat ist, das einen Anti-CD4-Antikörper umfasst, das an 40 % bis 100 % der CD4-Epitope davon gebunden ist.

9. Unmodifiziertes Transplantat zur Verwendung, modifiziertes Transplantat zur Verwendung oder Anti-CD4-Antikörper zur Verwendung nach Anspruch 8;
wobei das Inkubieren in Schritt a) des In-vitro-Verfahrens durchgeführt wird
i) für von 1 bis 150 Minuten, insbesondere für von 5 bis 150 Minuten, insbesondere für von 10 bis 150 Minuten, insbesondere für von 30 bis 150 Minuten, insbesondere für von 40 bis 120 Minuten, insbesondere für von 45 bis 90 Minuten, insbesondere für von 50 bis 70 Minuten;
oder
ii) für von 150 Minuten bis 7 Tagen, insbesondere für von 150 Minuten bis 5 Tagen, insbesondere von 150 Minuten bis 3 Tagen, insbesondere von 150 Minuten bis 1 Tag, insbesondere für von 150 Minuten bis 8 Stunden;
und/oder
wobei das Inkubieren in Schritt a) des *in* vitro-Verfahrens mit einer Antikörpermenge von 0,1 µg/ml bis 10 mg/ml durchgeführt wird,
und/oder
wobei das Inkubieren in Schritt a) des In-vitro-Verfahrens mit einer Antikörpermenge von 0,1µg/1 x 10⁹ kernhaltiger Zellen bis 100 mg/2 x 10⁶ kernhaltiger Zellen durchgeführt wird,
und/oder
wobei das Entfernen von ungebundenem Antikörpers von dem Transplantat in Schritt b) durch mindestens einmaliges Waschen des modifizierten Transplantats durchgeführt wird.

10. Unmodifiziertes Transplantat zur Verwendung, modifiziertes Transplantat zur Verwendung, oder Anti-CD4-Antikörper zur Verwendung nach einem beliebigen der vorangegangenen Ansprüche, wobei das Verfahren
i) eine Toleranz oder Teiltoleranz im Gewebe des Empfängers gegenüber dem unmodifizierten Transplantat impliziert;
und/oder
ii) eine verringerte Wahrscheinlichkeit für die Entwicklung von GvHD, von Donortransplantat-Abstoßung oder einer Organabstoßung, nach Transplantation des unmodifizierten Transplantats impliziert.

11. Unmodifiziertes Transplantat zur Verwendung, modifiziertes Transplantat zur Verwendung, oder Anti-CD4-Antikörper zur Verwendung nach einem beliebigen der vorangegangenen Ansprüche,
i) wobei das modifizierte Transplantat ausgewählt ist aus der Gruppe bestehend aus einer Zellsuspension enthaltend T-Zellen und Monozyten/Makrophagen die Zellsuspension umfassend Knochenmarkszellen, nicht-adhärente Knochenmarkszellen, periphere Blutzellen und/oder Nabelschnurblutzellen; und einer Zellsuspension umfassend Lymphozyten, Monozyten und/oder Makrophagen;;
und/oder
ii) wobei das Verfahren die Verabreichung einer Menge von 2 x 10⁶ Zellen bis 2 × 10¹⁵ kernhaltigen Zellen an das Subjekt umfasst.

12. Unmodifiziertes Transplantat zur Verwendung, modifiziertes Transplantat zur Verwendung oder Anti-CD4-Antikörper zur Verwendung nach einem beliebigen der vorangegangenen Ansprüche, wobei
A) das modifizierte Transplantat und das nicht modifizierte Transplantat von HLA-verwandten Donoren stammen;
insbesondere
i) wobei die HLA-verwandten Donoren insgesamt nicht mehr als 50 % HLA-fehlende Übereinstimmungen (mismatch), vorzugsweise nicht mehr als 40 % HLA-fehlende Übereinstimmungen (mismatch), vorzugsweise nicht mehr als 30 % HLA-fehlende Übereinstimmungen (mismatch), vorzugsweise nicht mehr als 20 % HLA-fehlende Übereinstimmungen (mismatch), vorzugsweise nicht mehr als 10 % HLA-fehlende Übereinstimmungen (mismatch), vorzugsweise keine HLA-fehlende Übereinstimmungen (mismatch) aufweisen;
und/oder
ii) wobei die HLA-verwandten Donoren aus der Gruppe ausgewählt sind, die aus gewebetyp-passenden Donoren, verwandten Zwillingen oder demselben Donor besteht;
insbesondere, wobei sowohl das modifizierte Transplantat als auch das unmodifizierte Transplantat von demselben Donor stammen,
und/oder
B) wobei das modifizierte Transplantat und das unmodifizierte Transplantat HLAverwandt sind;
wobei die HLA-verwandten Transplantate nicht mehr als insgesamt 50 % HLA-fehlende Übereinstimmungen (mismatch), vorzugsweise nicht mehr als 40 % HLA-fehlende Übereinstimmungen (mismatch), vorzugsweise nicht mehr als 30 % HLA- Fehlpaarungen, vorzugsweise nicht mehr als 20 % HLA-fehlende Übereinstimmungen (mismatch), vorzugsweise nicht mehr als 10 % HLA-fehlende Übereinstimmungen (mismatch), vorzugsweise keine HLA-fehlende Übereinstimmungen (mismatch) umfassen.

13. Unmodifiziertes Transplantat zur Verwendung, modifiziertes Transplantat zur Verwendung oder Anti-CD4-Antikörper zur Verwendung nach einem beliebigen der Ansprüche 3 bis 12, wobei der Anti-CD4-Antikörper
i ausgewählt ist aus der Gruppe bestehend aus Max16H5, OKT4A,OKTcdr4a, cMT-412, YHB.46, insbesondere wobei der Anti-CD4-Antikörper Max16H5 ist;
oder
ii) der Antikörper 30F16H5 ist;
oder
iii) erhältlich ist aus einer Zelllinie, die unter der Hinterlegungsnummer ECACC 88050502 hinterlegt ist;
oder
iv) erhältlich ist aus einer Zelllinie MAX.16H5/30F16H5, die am 2. Dezember 2011 beim DSMZ hinterlegt wurde;
oder
v) der Antikörper 16H5.chimIgG4 ist;
oder
vi) erhältlich ist aus einer Zelllinie CD4.16H5.chimIgG4, die am 2. Dezember 2011 beim DSMZ hinterlegt wurde;
oder
vii) ein Antikörper ist, der die VH und die VK des Antikörpers 16H5.chimIgG4 umfasst;
oder
viii) ein Antikörper ist, der eine VH und eine VK eines Antikörpers umfasst, der aus einer am 2. Dezember 2011 beim DSMZ hinterlegten Zelllinie CD4.16H5.chimIgG4 erhältlich ist;
oder
ix) ein Antikörper ist, der eine beliebige Kombination aus einer VH, ausgewählt aus SEQ ID NOs: 1-10, und einer VK, ausgewählt aus SEQ ID NOs: 11-20, umfasst, insbesondere wobei die Kombination aus VH1/VK1, VH2/VK2, VH4/VK2 und VH4/VK4 ausgewählt ist, besonders wobei die Kombination VH2/VK2 ist,
oder
x) eine Mischung von Antikörpern, ausgewählt aus den Antikörpern gemäß den vorstehenden i) bis ix) ist.

14. Unmodifiziertes Transplantat zur Verwendung, modifiziertes Transplantat zur Verwendung oder Anti-CD4-Antikörper zur Verwendung nach einem beliebigen der vorangegangenen Ansprüche,
wobei das unmodifizierte Transplantat und/oder das modifizierte Transplantat vor der Einführung in den Subjekt zusätzlich mit Wirkstoffen inkubiert wird/werden, die die Immunsuppression, Immuntoleranz und/oder die Bildung von regulatorischen T-Zellen fördern,
besonders mit einem Wirkstoff ausgewählt aus der Gruppe bestehend aus Il-2, TGF-β, Rapamycin, Retinsäure, 4-1BB-Ligand und Anti-CD28-Antikörpern; oder einer beliebigen Kombination davon.

15. Unmodifiziertes Transplantat zur Verwendung, modifiziertes Transplantat zur Verwendung oder Anti-CD4-Antikörper zur Verwendung nach einem beliebigen der vorangegangenen Ansprüche, wobei das modifizierte Transplantat verwendet wird
i) zum Erzielen von Toleranz oder Teiltoleranz im Gewebe des Empfängers gegenüber dem unveränderten Transplantat;
und/oder
ii) zum Verringern der Wahrscheinlichkeit des Auftretens von einem beliebigen aus der Gruppe bestehend aus GvHD, Donortransplantat-Abstoßung und Organabstoßung, nach Transplantation des unmodifizierten Transplantats.

## Revendications

1. Greffon non modifié pour utilisation dans un procédé de traitement d'une ou plusieurs maladies pouvant être traitées par transplantation chez un sujet,
ledit procédé comprenant une première étape d'introduction dans ledit sujet d'un greffon modifié, et une deuxième étape d'introduction dans ledit sujet dudit greffon non modifié,
ledit greffon modifié étant un greffon de suspension cellulaire contenant des cellules immunitaires, qui comprend un anticorps anti-CD4, lié à des épitopes CD4 dudit greffon de suspension cellulaire, et ledit greffon modifié pouvant être obtenu par un procédé in vitro comprenant les étapes suivantes :
a) incubation d'un greffon de suspension cellulaire contenant des cellules immunitaires avec l'anticorps anti-CD4, ladite incubation étant réalisée pendant 1 minute à 7 jours, et
b) élimination de l'anticorps anti-CD4 non lié dudit greffon, réduisant ainsi la quantité d'anticorps non lié dans ledit greffon,
et ledit greffon non modifié et ledit greffon modifié étant des greffons allogéniques et comprenant des cellules immunitaires portant l'antigène CD4.

2. Greffon modifié pour utilisation dans un procédé de traitement d'une ou plusieurs maladies pouvant être traitées par transplantation,
ledit procédé comprenant une première étape d'introduction dans ledit sujet dudit greffon modifié, et une deuxième étape d'introduction dans ledit sujet d'un greffon non modifié,
ledit greffon modifié étant un greffon de suspension cellulaire contenant des cellules immunitaires, qui comprend un anticorps anti-CD4, lié à des épitopes CD4 dudit greffon de suspension cellulaire, et ledit greffon modifié pouvant être obtenu par un procédé in vitro comprenant les étapes suivantes :
a) incubation d'un greffon de suspension cellulaire contenant des cellules immunitaires avec l'anticorps anti-CD4, ladite incubation étant réalisée pendant 1 minute à 7 jours, et
b) élimination de l'anticorps anti-CD4 non lié dudit greffon, réduisant ainsi la quantité d'anticorps non lié dans ledit greffon,
et ledit greffon non modifié et ledit greffon modifié étant des greffons allogéniques et comprenant des cellules immunitaires portant l'antigène CD4.

3. Anticorps anti-CD4, pour utilisation dans un procédé de traitement d'une ou plusieurs maladies pouvant être traitées par transplantation ;
ledit procédé comprenant une première étape d'introduction dans ledit sujet d'un greffon modifié, et une deuxième étape d'introduction dans ledit sujet d'un greffon non modifié,
ledit greffon modifié étant un greffon de suspension cellulaire contenant des cellules immunitaires, qui comprend un anticorps anti-CD4, lié à des épitopes CD4 dudit greffon de suspension cellulaire, et ledit greffon modifié pouvant être obtenu par un procédé in vitro comprenant les étapes suivantes :
a) incubation d'un greffon de suspension cellulaire contenant des cellules immunitaires avec l'anticorps anti-CD4, ladite incubation étant réalisée pendant 1 minute à 7 jours, et
b) élimination de l'anticorps anti-CD4 non lié dudit greffon, réduisant ainsi la quantité d'anticorps non lié dans ledit greffon,
et ledit greffon non modifié et ledit greffon modifié étant des greffons allogéniques et comprenant des cellules immunitaires portant l'antigène CD4.

4. Greffon non modifié, greffon modifié et/ou anticorps anti-CD4, pour utilisation dans
i) un procédé de transplantation chirurgicale,
ii) un procédé d'induction d'une tolérance à une transplantation, dans lequel un greffon modifié est utilisé pour induire une tolérance à la transplantation envers un greffon non modifié,
iii) un procédé de transfert d'une ou plusieurs suspensions cellulaires, en particulier une ou plusieurs suspensions cellulaires contenant des cellules souches, d'un donneur à un sujet,
iv) un procédé de transfert d'un ou plusieurs tissus d'un donneur à un sujet,
v) un procédé de transfert d'un ou plusieurs organes partiels d'un donneur à un sujet,
vi) un procédé de transfert d'un ou plusieurs organes d'un donneur à un sujet,
vii) un procédé de transfert de cellules reconstituant le système hématopoïétique d'une source donneuse vers un sujet, éventuellement suivi du transfert d'un ou plusieurs organes, depuis ladite source donneuse vers ledit sujet,
ou
viii) un procédé d'amélioration de la reconstitution immunitaire chez un sujet, éventuellement suivie du transfert d'un ou plusieurs organes chez ledit sujet ;
ledit procédé comprenant une première étape d'introduction dans ledit sujet d'un greffon modifié, et une deuxième étape d'introduction dans ledit sujet d'un greffon non modifié,
ledit greffon modifié étant un greffon de suspension cellulaire contenant des cellules immunitaires, qui comprend un anticorps anti-CD4, lié à des épitopes CD4 dudit greffon de suspension cellulaire, et ledit greffon modifié pouvant être obtenu par un procédé in vitro comprenant les étapes suivantes :
a) incubation d'un greffon de suspension cellulaire contenant des cellules immunitaires avec l'anticorps anti-CD4, ladite incubation étant réalisée pendant 1 minute à 7 jours, et
b) élimination de l'anticorps anti-CD4 non lié dudit greffon,
et ledit greffon non modifié et ledit greffon modifié étant des greffons allogéniques et comprenant des cellules immunitaires portant l'antigène CD4.

5. Greffon non modifié pour utilisation, greffon modifié pour utilisation, ou anticorps anti-CD4 pour utilisation selon l'une quelconque des revendications précédentes,
ladite première étape étant réalisée pendant 30 min à 50 ans, notamment d'une heure à deux mois avant ladite deuxième étape,
ou
ladite première étape étant réalisée au moins une heure, en particulier au moins trois semaines avant ladite deuxième étape.

6. Greffon non modifié pour utilisation, greffon modifié pour utilisation ou anticorps anti-CD4 pour utilisation selon l'une quelconque des revendications précédentes, ledit greffon modifié comprenant des cellules souches.

7. Greffon non modifié pour utilisation, greffon modifié pour utilisation, ou anticorps anti-CD4 pour utilisation selon l'une quelconque des revendications précédentes,
i) ledit greffon non modifié étant une suspension cellulaire,
ou
ii) ledit greffon non modifié étant un tissu,
ou
iii) ledit greffon non modifié étant un organe.

8. Greffon non modifié pour utilisation, greffon modifié pour utilisation, ou anticorps anti-CD4 pour utilisation selon l'une quelconque des revendications précédentes, ledit greffon modifié étant un greffon de suspension cellulaire contenant des cellules immunitaires qui comprend un anticorps anti-CD4, lié à 40 %. à 100 % de leurs épitopes CD4.

9. Greffon non modifié pour utilisation, greffon modifié pour utilisation ou anticorps anti-CD4 pour utilisation selon la revendication 8 ;
ladite incubation à l'étape a) dudit procédé in vitro est réalisée
i) pendant 1 à 150 minutes, particulièrement pendant 5 minutes à 150 minutes, plus particulièrement pendant 10 minutes à 150 minutes, plus particulièrement pendant 30 minutes à 150 minutes, plus particulièrement pendant 40 minutes à 120 minutes, plus particulièrement pendant 45 minutes à 90 minutes, notamment pendant 50 minutes à 70 minutes ;
ou
ii) pendant 150 minutes à 7 jours, notamment pendant 150 minutes à 5 jours, plus particulièrement de 150 minutes à 3 jours, plus particulièrement de 150 minutes à 1 jour, notamment de 150 minutes à 8 heures ;
et/ou
ladite incubation à l'étape a) dudit procédé in vitro étant réalisée avec une quantité d'anticorps de 0,1 µg/ml à 10 mg/ml, et/ou
ladite incubation à l'étape a) dudit procédé in vitro étant réalisée avec une quantité d'anticorps de 0,1 µg/1 × 10⁹ cellules nucléées à 100 mg/2 x 10⁶ cellules nucléées, et/ou
ladite élimination de l'anticorps non lié dudit greffon à l'étape b) étant réalisée par lavage du greffon modifié au moins une fois.

10. Greffon non modifié pour utilisation, greffon modifié pour utilisation ou anticorps anti-CD4 pour utilisation selon l'une quelconque des revendications précédentes, ledit procédé
i) impliquant une tolérance ou une tolérance partielle dans le tissu du receveur audit greffon non modifié ;
et/ou
ii) impliquant une probabilité réduite de développement de l'un quelconque du groupe comprenant une réaction de greffon contre l'hôte, un rejet de greffon du donneur et un rejet d'organe, lors de la transplantation du greffon non modifié.

11. Greffon non modifié pour utilisation, greffon modifié pour utilisation, ou anticorps anti-CD4 pour utilisation selon l'une quelconque des revendications précédentes,
i) ledit greffon modifié étant choisi dans le groupe constitué d'une suspension cellulaire contenant des lymphocytes T et des monocytes/macrophages, la suspension cellulaire comprenant des cellules de moelle osseuse, des cellules de moelle osseuse non adhérentes, des cellules de sang périphérique et/ou des cellules de sang ombilical ; et une suspension cellulaire comprenant des lymphocytes, des monocytes et/ou des macrophages ;
et/ou
ii) ledit procédé comprenant l'administration d'une quantité allant de 2 × 10⁶ cellules à 2 × 10¹⁵ cellules nucléées audit sujet.

12. Greffon non modifié pour utilisation, greffon modifié pour utilisation ou anticorps anti-CD4 pour utilisation selon l'une quelconque des revendications précédentes,
A) le greffon modifié et le greffon non modifié provenant de donneurs HLA compatibles ; en particulier
i) lesdits donneurs HLA compatibles ne comprenant pas plus d'un total de 50 % de discordances HLA, de préférence pas plus de 40 % de discordances HLA, de préférence pas plus de 30 % de discordances HLA, de préférence pas plus de 20 % de discordances HLA, de préférence pas plus de 10 % de discordances HLA, de préférence aucune discordance HLA ;
et/ou
ii) dans lequel lesdits donneurs apparentés à HLA sont sélectionnés dans le groupe constitué de donneurs appariés au type de tissu, de jumeaux apparentés ou sont le même donneur ;
en particulier dans lequel le greffon modifié et le greffon non modifié proviennent du même donneur,
et/ou
B) dans lequel ledit greffon modifié et le greffon non modifié sont liés à HLA ; en particulier dans lequel lesdites greffes liées à HLA ne comprennent pas plus d'un total de 50 % de discordances HLA, de préférence pas plus de 40 % de discordances HLA, de préférence pas plus de 30 % de discordances HLA, de préférence pas plus de 20 % de discordances HLA, de préférence pas plus plus de 10 % de discordances HLA, de préférence aucune discordance HLA.

13. Greffon non modifié pour utilisation, greffon modifié pour utilisation, ou anticorps anti-CD4 pour utilisation selon l'une quelconque des revendications 3 à 12, ledit anticorps anti-CD4
i) étant choisi dans le groupe constitué de Max16H5, OKT4A, OKTcdr4a, cMT-412, YHB.46, ledit anticorps anti-CD4 étant notamment Max16H5 ;
ou
ii) étant l'anticorps 30F16H5 ;
ou
iii) pouvant être obtenu à partir d'une lignée cellulaire déposée sous le numéro d'accession ECACC 88050502 ;
ou
iv) pouvant être obtenu à partir d'une lignée cellulaire MAX.16H5/30F16H5 déposée auprès du DSMZ le 2 décembre 2011 ;
ou
v) étant l'anticorps 16H5.chimlgG4 ;
ou
vi) pouvant être obtenu à partir d'une lignée cellulaire CD4.16H5.chimlgG4 déposée auprès du DSMZ le 2 décembre 2011 ;
ou
vii) étant un anticorps comprenant le VH et le VK de l'anticorps 16H5.chimlgG4 ; ou
viii) étant un anticorps comprenant un VH et un VK d'un anticorps pouvant être obtenu à partir d'une lignée cellulaire CD4.16H5.chimlgG4 déposée auprès du DSMZ le 2 décembre 2011 ;
ou
ix) étant un anticorps comprenant une combinaison quelconque d'un VH choisi parmi les SEQ ID NO : 1 à 10 et d'un VK choisi parmi les SEQ ID NO : 11 à 20, en particulier dans lequel ladite combinaison est choisie parmi VH1/VK1, VH2/VK2, VH4/VK2 et VH4/VK4, ladite combinaison étant notamment VH2/VK2,
ou
x) étant un mélange d'anticorps choisis parmi les anticorps selon i) à ix) ci-dessus.

14. Greffon non modifié pour utilisation, greffon modifié pour utilisation, ou anticorps anti-CD4 pour utilisation selon l'une quelconque des revendications précédentes,
où, avant introduction dans ledit sujet, ledit greffon non modifié et/ou le greffon modifié sont en outre incubés avec des agents favorisant l'immunosuppression, l'immunotolérance et/ou la formation de lymphocytes T régulateurs, en particulier avec un agent choisi dans le groupe constitué de II-2 , TGF-β, la rapamycine, l'acide rétinoïque, le ligand 4-1BB et des anticorps anti-CD28 ; ou une combinaison quelconque de ceux-ci.

15. Greffon non modifié pour utilisation, greffon modifié pour utilisation ou anticorps anti-CD4 pour utilisation selon l'une quelconque des revendications précédentes, le greffon modifié étant utilisé
i) pour obtenir une tolérance ou une tolérance partielle dans le tissu du receveur au greffon non modifié ;
et/ou
ii) pour réduire la probabilité de l'un quelconque du groupe constitués d'une réaction de greffon contre l'hôte, un rejet de greffon du donneur et un rejet d'organe, lors de la transplantation dudit greffon non modifié.
